# EUROPEAN PATENT APPLICATION

(11) **EP 3 360 953 A1**
(43) Date of publication of application: **15.08.2018**
(21) Application number: 16853771.0
(22) Date of filing: 07.10.2016
(51) Int. Cl.: C12M 1/00, C12M 1/34, C12M 1/42, C12Q 1/68, G01N 37/00

(54) **ANALYTE TREATMENT CHIP, ANALYTE TREATMENT DEVICE, AND ANALYTE TREATMENT METHOD**

(30) Priority: 09.10.2015 JP 2015200770
(71) Applicant: Sysmex Corporation, Kobe-shi, Hyogo 651-0073 (JP)
(72) Inventor: TAGAWA, Ayato, Kobe-shi Hyogo 651-0073 (JP); NAKANO, Tsuyoshi, Kobe-shi Hyogo 651-0073 (JP); KAWAMOTO, Yasuko, Kobe-shi Hyogo 651-0088 (JP); YAMAWAKI, Koya, Kobe-shi Hyogo 651-0073 (JP); TOBIMATSU, Hiroaki, Kobe-shi Hyogo 651-0073 (JP)
(74) Representative: Hoffmann Eitle
(86) International application number: PCT/JP2016/080009
(87) International publication number: WO 2017/061620

(57) **Abstract**

The present specimen treatment chip includes: a first flow channel for forming a droplet containing a mixed liquid of a nucleic acid, a reagent for an amplification reaction of the nucleic acid, and a carrier to which a primer for binding to the nucleic acid is added, in a dispersion medium; a second flow channel for amplifying the nucleic acid in the droplet; and a third flow channel for mixing the droplet containing the carrier with the primer having bound to an amplification product of the nucleic acid, and a reagent for breaking down the droplet, to break down the droplet.

## Description

### TECHNICAL FIELD

There is a technique for detecting a nucleic acid using a specimen treatment chip (e.g., refer to Patent Literature 1).

### BACKGROUND ART

Patent Literature 1 above discloses a specimen treatment apparatus including a PCR unit having a circulation flow channel, a droplet forming unit, and a detection unit. The droplet forming unit injects a mixed liquid containing a nucleic acid and a PCR reagent into a carrier liquid flowing through the circulation flow channel. The carrier liquid is immiscible to the mixed liquid, and a droplet of the mixed liquid is formed in the flow of the carrier liquid. The PCR unit amplifies the nucleic acid in the droplet while circulating the droplet. The amplified nucleic acid is detected by the detection unit while being contained in the droplet flowing through the circulation flow channel.

### CITATION LIST

Patent Literature 1: U.S. Reissue Patent No. 43365

### SUMMARY OF INVENTION

### TECHNICAL PROBLEMS

In Patent Literature 1 above, a nucleic acid is detected while being contained in a droplet, so that it is required to accurately control a particle diameter of the droplet, and generation speed of the droplet in the circulation flow channel. Thus, it is desired to treat a specimen so that a nucleic acid can be detected without requiring accurate control of a particle diameter of a droplet, generation speed thereof, and the like.

The present invention is directed to treatment of a specimen so that a nucleic acid can be detected without requiring accurate control of a particle diameter of a droplet, generation speed thereof, and the like.

### SOLUTIONS TO PROBLEMS

A specimen treatment chip according to a first aspect of the present invention is installed in a specimen treatment apparatus to treat a nucleic acid in a specimen supplied by the specimen treatment apparatus, and the specimen treatment chip includes: a first flow channel for forming a droplet containing a mixed liquid of a nucleic acid, a reagent for an amplification reaction of the nucleic acid, and a carrier to which a primer for binding to the nucleic acid is added, in a dispersion medium; a second flow channel for amplifying the nucleic acid in the droplet; and a third flow channel for mixing the droplet containing the carrier with the primer binding to an amplification product of the nucleic acid, and a reagent for breaking down the droplet, to break down the droplet, wherein the carrier extracted from the droplet broken down is collected to cause the amplified product on the collected carrier and a marking substance for detecting the amplification product to react with each other.

A specimen treatment apparatus according to a second aspect of the present invention is configured to treat a nucleic acid being a specimen by using the specimen treatment chip according to the first aspect, and includes: an installation unit that installs a specimen treatment chip; a liquid feeder for supplying and feeding a liquid containing the nucleic acid to the specimen treatment chip; and a control unit for controlling the liquid feeder so as to cause a liquid containing the nucleic acid to be fed in the specimen treatment chip while flowing through the first flow channel, the second flow channel, and the third flow channel, of the specimen treatment chip, in order.

A specimen treatment method according to a third aspect of the present invention is configured to treat a nucleic acid being a specimen by using a specimen treatment chip having a first flow channel, a second flow channel, and a third flow channel, and the specimen treatment method includes the steps of: supplying a mixed liquid of the nucleic acid, a reagent for an amplification reaction of the nucleic acid, and a carrier to which a primer for binding to the nucleic acid is added, and a dispersion medium, to the first flow channel to form a droplet; supplying an emulsion containing the droplet to the second flow channel to amplify the nucleic acid; supplying the droplet containing the carrier with the primer binding to an amplification product of the nucleic acid, and a reagent for breaking down the droplet, to the third flow channel to break down the droplet; and collecting the carrier extracted from the droplet broken down to cause the amplification product on the collected carrier and a marking substance for detecting the amplification product to react with each other.

A specimen treatment chip according to a fourth aspect of the present invention is configured to be installed in a specimen treatment apparatus to treat a nucleic acid in a specimen, and includes a breaking-down flow channel for mixing a droplet containing a carrier binding to an amplification product of the nucleic acid, amplified in the droplet, and a reagent for breaking down a droplet, to break down the droplet.

A specimen treatment apparatus according to a fifth aspect of the present invention is configured to treat a nucleic acid in a specimen by using the specimen treatment chip according to the fourth aspect, and includes: an installation unit that installs a specimen treatment chip; a liquid feeder for supplying and feeding a liquid containing the nucleic acid to the specimen treatment chip; and a control unit for controlling the liquid feeder so as to cause the liquid containing the nucleic acid to be fed in the specimen treatment chip while flowing through the breaking-down flow channel of the specimen treatment chip.

A specimen treatment method according to a sixth aspect of the present invention is configured to treat a nucleic acid by using a specimen treatment chip, and the specimen treatment method includes the steps of: forming a droplet containing a mixed liquid of the nucleic acid, a reagent for an amplification reaction of the nucleic acid, and a carrier to which a primer for binding to the nucleic acid is added, in a dispersion medium; amplifying the nucleic acid in the droplet; and supplying the droplet containing the carrier with the primer binding to an amplification product of the nucleic acid, and a reagent for breaking down the droplet, to the breaking-down flow channel provided the specimen treatment chip to break down the droplet.

### ADVANTAGEOUS EFFECTS OF INVENTION

It is possible to treat a specimen without requiring accurate control of a particle diameter of a droplet, generation speed thereof, and the like.

### BRIEF DESCRIPTION OF DRAWINGS

Fig. 1 is a diagram for illustrating an outline of a specimen treatment chip.
Fig. 2 is a diagram for illustrating an outline of a specimen treatment apparatus.
Fig. 3 is a perspective view illustrating a configuration example of a specimen treatment chip.
Fig. 4 is a plan view illustrating a configuration example of a substrate of a specimen treatment chip.
Fig. 5 is a plan view illustrating a configuration example of a fluid module.
Fig. 6 is a schematic plan view illustrating an example of placement of fluid modules on a substrate.
Fig. 7 is a schematic longitudinal sectional view illustrating an example of placement of a fluid module on a substrate.
Fig. 8 is a longitudinal sectional view illustrating a first modification of a specimen treatment chip.
Fig. 9 is a longitudinal sectional view illustrating a second modification of a specimen treatment chip.
Fig. 10 is a block diagram illustrating a configuration example of a specimen treatment apparatus.
Fig. 11 is a sectional view illustrating a configuration example of a valve.
Fig. 12 is a longitudinal sectional view illustrating a configuration example of a liquid reservoir.
Fig. 13 is a longitudinal sectional view illustrating a first configuration example of a lid for a liquid reservoir.
Fig. 14 is a longitudinal sectional view illustrating a second configuration example of a lid for a liquid reservoir.
Fig. 15 is a longitudinal sectional view illustrating a first configuration example of a lid of an installation unit.
Fig. 16 is a longitudinal sectional view illustrating a second configuration example of a lid of an installation unit.
Fig. 17 is a longitudinal sectional view illustrating a first configuration example of a connector.
Fig. 18 is a longitudinal sectional view illustrating a second configuration example of a connector.
Fig. 19 is a schematic diagram illustrating a third configuration example of a connector.
Fig. 20 is an exploded view illustrating a configuration example of a fixture.
Fig. 21 illustrates a fixture in a state where a specimen treatment chip is fixed.
Fig. 22 includes a top view (A) and a bottom view (B) of the fixture in Fig. 21.
Fig. 23 is a schematic diagram illustrating an installation example of various units.
Fig. 24 includes a bottom view (A) illustrating a placement example of a heater unit in a fixture and a schematic sectional view (B) illustrating a placement example of a heater unit in an installation unit.
Fig. 25 includes a top view (A) illustrating a placement example of a detection unit in a fixture and a schematic sectional view (B) illustrating a placement example of a detection unit in an installation unit.
Fig. 26 includes a bottom view (A) illustrating a placement example of a magnet unit in a fixture and a schematic sectional view (B) illustrating a placement example of a detection unit in an installation unit.
Fig. 27 is a flowchart illustrating an example of opening and closing control of a valve by a control unit.
Fig. 28 is a flowchart illustrating an example of control of opening-closing timing of a valve by a control unit.
Fig. 29 is a flowchart illustrating an example of storage processing of a liquid into a liquid reservoir by a control unit.
Fig. 30 is a flowchart illustrating an example of an emulsion PCR assay.
Fig. 31 illustrates progress of reaction in an emulsion PCR assay.
Fig. 32 illustrates a configuration example of a specimen treatment chip used in an emulsion PCR assay.
Fig. 33 illustrates a configuration example of a sixth flow channel.
Fig. 34 illustrates a configuration example of a first flow channel.
Fig. 35 is an enlarged view illustrating a first example of an intersection where an emulsion is formed.
Fig. 36 is an enlarged view illustrating a second example of an intersection where an emulsion is formed.
Fig. 37 illustrates a configuration example of a second flow channel.
Fig. 38 illustrates a configuration example of a third flow channel.
Fig. 39 illustrates a configuration example of a fourth flow channel.
Fig. 40 illustrates an example of operation of cleaning and concentrating magnetic particles in the fourth flow channel.
Fig. 41 illustrates a configuration example of a fifth flow channel.
Fig. 42 is a view for illustrating a specimen treatment chip having a breaking-down flow channel.
Fig. 43 is a view for illustrating a specimen treatment chip having a breaking-down flow channel and a marking flow channel.
Fig. 44 illustrates a configuration example of a specimen treatment chip having a breaking-down flow channel and a marking flow channel.
Fig. 45 illustrates first examples (A) and (B), and second examples (C) and (D), of a structure for promoting mixing of liquids.
Fig. 46 is a longitudinal sectional view illustrating a configuration example of a specimen treatment chip provided with a reservoir.
Fig. 47 is a block diagram illustrating a configuration example of a specimen treatment apparatus.
Fig. 48 is a longitudinal sectional view illustrating a connector connected to a specimen treatment chip.
Fig. 49 is a perspective view illustrating a configuration example of a specimen treatment apparatus.
Fig. 50 is a diagram illustrating an example of performing an emulsion PCR assay using a plurality of specimen treatment chips.

### DESCRIPTION OF EMBODIMENTS

Hereinafter, embodiments will be described with reference to the drawings.

### [Overview of specimen treatment chip]

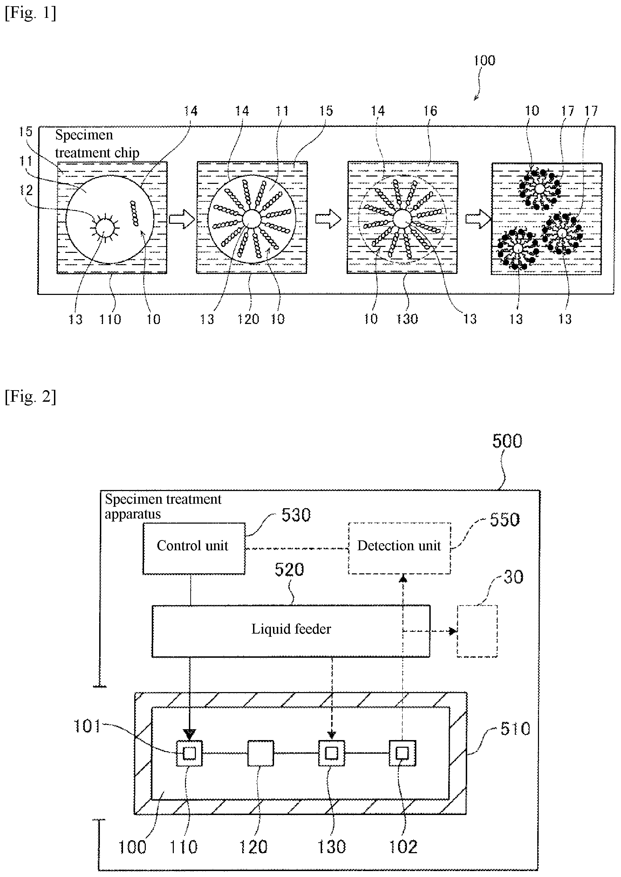

With reference to Fig. 1, an overview of a specimen treatment chip according to the present embodiment will be described.

A specimen treatment chip 100 according to the present embodiment is a chip installed in a specimen treatment apparatus 500 (refer to Fig. 2) to treat an object component in a specimen supplied by the specimen treatment apparatus 500 in a plurality of steps. The specimen treatment chip 100 is a specimen treatment chip of a cartridge type that is configured to be capable of receiving a liquid containing an object component, and that is set in the specimen treatment apparatus 500 to enable the specimen treatment apparatus 500 to perform specimen treatment. In addition, the specimen treatment chip 100 is a microfluidic chip in which a fine flow channel for performing a desired treatment step is formed, as described later. The flow channel is a microchannel having a sectional dimension (width, height, and inner diameter) of 0.1 µm to 1000 µm, for example.

The specimen treatment chip 100 is configured to perform predetermined specimen treatment for performing genetic testing using a nucleic acid such as DNA (deoxyribonucleic acid) as an object component. Into the specimen treatment chip 100, a liquid such as body fluid or blood (whole blood, serum, or plasma) collected from a patient, or a liquid obtained by applying predetermined pretreatment to collected body fluid or blood, is injected as a specimen. For example, an extraction liquid in which a nucleic acid is extracted from blood or the like by predetermined pretreatment is injected into the specimen treatment chip 100. A nucleic acid may be extracted inside the specimen treatment chip 100.

The liquid containing a nucleic acid injected into the specimen treatment chip 100 is fed into the specimen treatment chip 100 by the specimen treatment apparatus 500. In the course of feeding the liquid containing the nucleic acid, treatment in a plurality of steps is performed in a predetermined order. As a result of the plurality of treatment steps, a measurement sample suitable for detecting the nucleic acid is generated in the specimen treatment chip 100.

The specimen treatment chip 100 includes a first flow channel 110, a second flow channel 120, and a third flow channel 130. The first flow channel 110, the second flow channel 120, and the third flow channel 130 are disposed in series so that the liquid containing a nucleic acid 10 is supplied in this order. Another flow channel may be interposed among the first flow channel 110, the second flow channel 120, and the third flow channel 130.

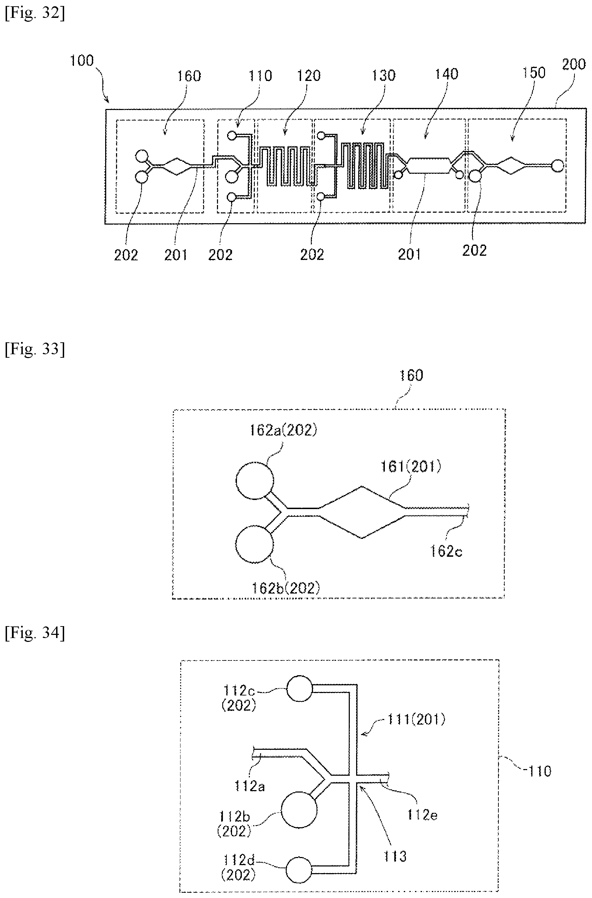

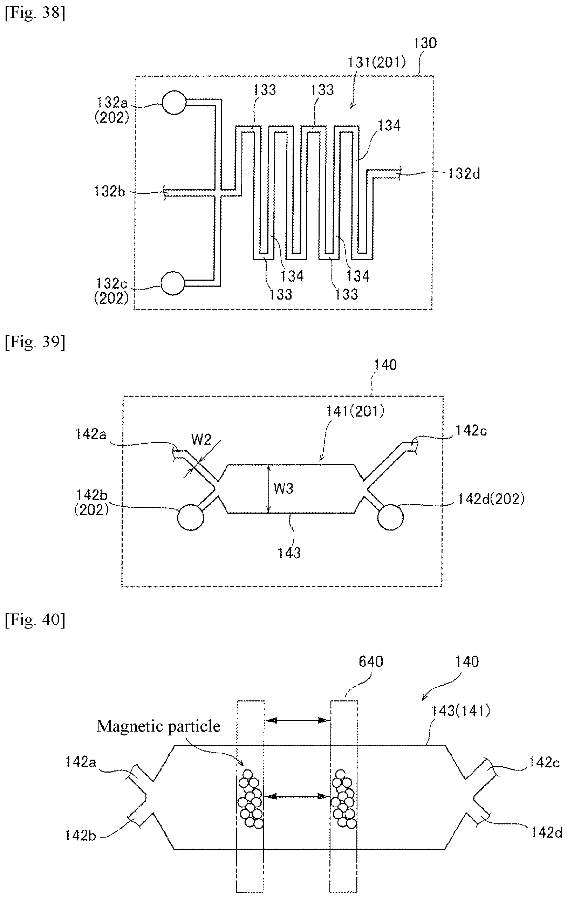

The flow channel of the specimen treatment chip 100 may have any structure as long as it allows a liquid injected from an inlet portion of the specimen treatment chip 100 to flow. The flow channel has a shape suitable for treatment to be performed in the flow channel. The flow channel is formed so as to have a flow channel width, a flow channel height or a flow channel depth, a flow channel length, and a volume, suitable for treatment to be performed in the flow channel. The flow channel is composed of an elongated tubular passage or channel, for example. The channel can be formed in a linear shape, a curved shape, a zigzag shape, or the like. As described later, the flow channel may have a shape (refer to Fig. 33) in which dimensions of the flow channel, such as width and height of the flow channel, change, a shape in which a part or all of the flow channel expands in a planar shape (refer to Fig. 39), a chamber shape (not illustrated) capable of storing an inflowing liquid, or the like.

The first flow channel 110 is used to form, in a dispersion medium 15, droplets 14 each containing a mixed liquid of a nucleic acid 10 as an object component, a reagent 11 for an amplification reaction of the nucleic acid 10, and a carrier 13 to which a primer 12 for binding to the nucleic acid 10 is added. For example, into the first flow channel 110, a liquid containing the nucleic acid 10, the reagent 11 for the amplification reaction, and a liquid containing the carrier 13 are supplied, and then are mixed in the first flow channel 110. These kinds of liquid may be supplied into the first flow channel 110 in a mixed liquid state. The reagent 11 for an amplification reaction contains a substance necessary for polymerase chain reaction (PCR), such as DNA polymerase. As the carrier 13, a nonmagnetic particle or a magnetic particle is available.

In addition, the dispersion medium 15 is supplied into the first flow channel 110. For example, the mixed liquid is water-based, and the dispersion medium 15 is oil-based. As the dispersion medium 15, a liquid such as oil having immiscibility with the mixed liquid is used, for example. For example, when the mixed liquid is supplied from a direction intersecting with a flow direction of the dispersion medium 15 to cause a flow of the mixed liquid to be sheared by a flow of the dispersion medium 15, each of the droplets 14 of the mixed liquid is formed in the dispersion medium 15. Each of the droplets 14 may be formed by intermittently supplying a minute amount of the mixed liquid into a flow of the dispersion medium 15. In the first flow channel 110, an emulsion is formed in which the droplets 14 of the mixed liquid are dispersed in the dispersion medium 15. An emulsion is a dispersion solution in which a liquid not mixed with the dispersion medium is dispersed in the dispersion medium. Thus, the emulsion contains the dispersion medium 15 and a large number of the droplets 14 dispersed in the dispersion medium 15.

The second flow channel 120 is used to amplify the nucleic acid 10 in the droplet 14 formed in the first flow channel 110. For example, a thermal cycle is performed in the second flow channel 120 so that the nucleic acid 10 is amplified.

For example, a plurality of temperature zones suitable for amplification treatment is formed in a path of the second flow channel 120, and an emulsion containing the droplets 14 passes through each of the temperature zones. In addition, the emulsion containing the droplets 14 is temporarily stored at a heating position set in the second flow channel 120, and temperature at the heating position is periodically changed, for example. As a result, an amplification product of the nucleic acid 10 is formed in each of the droplets 14 in the second flow channel 120. The amplified nucleic acid 10 binds to the carrier 13 through the primer 12 in the droplet 14. The specimen treatment apparatus 500 (refer to Fig. 2) forms the temperature zones in the second flow channel 120 and performs periodic temperature control.

The third flow channel 130 is used to mix the droplet 14 containing the carrier 13 with the primer 12 binding to the amplification product of the nucleic acid 10 and a reagent 16 for breaking down the droplet 14 to break down the droplet 14. The reagent 16 for breaking down the droplet 14 contains alcohol, a surfactant, or the like, for example. Into the third flow channel 130, the emulsion containing the droplets 14 and the reagent 16 for breaking down the droplet 14 are supplied. When the emulsion and the reagent 16 for breaking down the droplet 14 are mixed, the droplet 14 in the emulsion is broken down (indicated by a broken line). As a result, the carrier 13 binding to the amplification product of the nucleic acid 10 is extracted from each of the droplets 14 into the dispersion medium 15 in the third flow channel 130.

The specimen treatment chip 100 is configured to collect the carrier 13 extracted from the droplet 14 by breaking down the droplet 14 and to cause the amplified product on the collected carrier 13 to react with a marking substance 17 for detecting an amplification product.

A step of collecting the carrier 13 extracted from the droplet 14 by breaking down the droplet 14 and a step of causing the amplified product on the collected carrier 13 to react with the marking substance 17 may be performed in a common flow channel, or flow channels for performing the respective steps may be separately provided in the specimen treatment chip 100. These steps also may be performed in the third flow channel 130, or in the first flow channel 110 and the second flow channel 120 by returning the liquid.

In the step of collecting the carrier 13 extracted from the droplet 14 by breaking down the droplet 14, cleaning treatment for removing unnecessary components is performed while the carrier 13 is collected, for example. The cleaning treatment is performed by a method for disposing the collected carrier 13 in a flow of a cleaning liquid, for example. This enables the dispersion medium 15 such as oil to be removed from the carrier 13. When a magnetic particle is used as the carrier 13, the carrier 13 can be collected by a magnetic force.

The step of causing the amplified product on the collected carrier 13 to react with the marking substance 17 is performed by hybridization of the nucleic acid 10 and the marking substance 17. The marking substance 17 is designed to specifically bind to the DNA to be detected. The marking substance 17 is a substance that emits fluorescence as an optical signal, for example. In this case, a nucleic acid can be detected on the basis of a mark by a flow cytometry method or the like in which fluorescence generated by laser irradiation is detected with a detector.

The specimen treatment chip 100 includes a port 101 (refer to Fig. 2) for supplying a liquid to each flow channel, for example. The specimen treatment chip 100 is configured to feed a liquid in the first flow channel 110, the second flow channel 120, and the third flow channel 130 by using pressure supplied from the specimen treatment apparatus 500 through a port 101 for injecting a specimen, or the like. That is, the specimen treatment chip 100 is configured so as to operate in cooperation with the specimen treatment apparatus 500. This eliminates the need to provide a structure for feeding liquid to a specimen treatment chip 100 side, so that the specimen treatment chip 100 can be downsized.

In addition, the specimen treatment chip 100 includes a port 102 (refer to Fig. 2) for recovering a liquid having passed through the first flow channel 110, the second flow channel 120, and the third flow channel 130, for example. Providing the port 102 for recovering a liquid enables a liquid containing a marked nucleic acid 10 to be easily extracted when a nucleic acid is detected outside the specimen treatment chip 100.

The above configuration enables the nucleic acid 10 held on a carrier 13 to be extracted from a droplet 14 by breaking down the droplet 14 after amplification treatment in the third flow channel 130 in the specimen treatment chip 100 of the present embodiment. Then, the carrier 13 extracted from the droplet 14 by breaking down the droplet 14 is collected and an amplified product on the collected carrier 13 is caused to react with a marking substance 17, so that the nucleic acid 10 extracted from each of the droplets 14 can be detected. As a result, unlike the case where the nucleic acid 10 is detected while being contained in the droplet 14, it is possible to treat a specimen in the specimen treatment chip 100 without requiring accurate control of a particle diameter of the droplet 14, generation speed thereof, and the like.

### [Overview of specimen treatment apparatus]

Next, an overview of a specimen treatment apparatus according to the present embodiment will be described with reference to Fig. 2.

A specimen treatment apparatus 500 is configured to treat a nucleic acid 10 being an object component using a specimen treatment chip 100.

The specimen treatment apparatus 500 includes an installation unit 510 for installing the specimen treatment chip 100, a liquid feeder 520, and a control unit 530 for controlling the liquid feeder 520.

The installation unit 510 is formed in a shape corresponding to the specimen treatment chip 100 to support the specimen treatment chip 100. The installation unit 510 has a structure such that at least one of an upper side and a lower side of the specimen treatment chip 100 is opened to be connected to a flow channel of the specimen treatment chip 100 and to allow a unit used for various treatment steps in the specimen treatment chip 100 to be installed.

The liquid feeder 520 has a function of supplying and feeding a liquid containing a nucleic acid 10 to the specimen treatment chip 100. The liquid feeder 520 is composed of a combination of a pump and a valve, for example, and feeds a liquid in the specimen treatment chip 100 under pressure. The liquid feeder 520 supplies not only the liquid containing the nucleic acid but also various reagents to be used in the specimen treatment chip 100 to the specimen treatment chip 100, for example. The liquid feeder 520 is connected to a reservoir for storing the liquid containing the nucleic acid and a reservoir for storing various reagents, and supplies the liquid and the reagents, for example.

In addition, the liquid feeder 520 can advance liquid in the specimen treatment chip 100 according to the order of steps by supplying positive pressure, and can discharge the liquid from the specimen treatment chip 100. The liquid feeder 520 may feed and discharge the liquid of the specimen treatment chip 100 by supplying negative pressure.

The control unit 530 controls the liquid feeder 520 so as to cause the liquid containing the nucleic acid 10 to be fed in the specimen treatment chip 100 while flowing through the first flow channel 110, the second flow channel 120, and the third flow channel 130, of the specimen treatment chip 100, in order.

The liquid feeder 520 is controlled by controlling supply pressure of the liquid feeder 520 with a flow sensor or a pressure sensor provided in a liquid supply path, for example. When a metering pump, such as a syringe pump and a diaphragm pump, is used as the liquid feeder 520, a flow rate sensor is not necessarily required.

When treatment units used for various respective treatment steps are installed in the specimen treatment apparatus 500, the control unit 530 may control these treatment units. The treatment units used for various treatment steps include a heater unit or a cooling unit for controlling temperature of a liquid in the second flow channel 120, a magnet unit for applying a magnetic force to the liquid, and a camera unit for imaging the liquid, for example. These treatment units are provided corresponding to at least one of the plurality of flow channels, and are configured to operate when the respective treatment steps are performed in the corresponding flow channels.

The specimen treatment apparatus 500 includes a detection unit 550 for detecting a nucleic acid on the basis of a mark caused by a marking substance 17 binding to an amplification product, for example. The detection unit 550 is a flow cytometer, for example. In this case, the liquid feeder 520 may be configured so as to recover a liquid containing the marked nucleic acid from the specimen treatment chip 100 to supply the liquid to the detection unit 550. This enables not only specimen treatment for sample preparation using the specimen treatment chip 100, but also nucleic acid detection, in the specimen treatment apparatus 500, so that convenience of the apparatus is improved.

The specimen treatment apparatus 500 may not include the detection unit 550. That is, nucleic acid detection may be performed by a detection device (not illustrated) outside the specimen treatment apparatus 500. In that case, the liquid feeder 520 recovers a liquid containing a marked nucleic acid 10 from the specimen treatment chip 100 and dispenses the liquid to a sample container 30 for feeding, or the like, or the liquid feeder 520 and an external detection device are connected by a liquid feeding member such as a tube, for example.

The specimen treatment apparatus 500 performs a series of specimen treatment for nucleic acid detection using the specimen treatment chip 100. That is, the specimen treatment apparatus 500 supplies a mixed liquid of a nucleic acid 10, a reagent 11 for an amplification reaction and a carrier 13 to which a primer 12 is added, and a dispersion medium 15 to the first flow channel 110 to form droplets 14. The specimen treatment apparatus 500 supplies an emulsion containing the droplets 14 to the second flow channel 120 to amplify the nucleic acid 10. The specimen treatment apparatus 500 supplies the droplets 14 each including the carrier 13 with the primer 12 binding to an amplification product of the nucleic acid 10 and the reagent 16 for breaking down the droplets 14 to the third flow channel 130 to break down the droplets 14. The specimen treatment apparatus 500 collects the carrier 13 extracted from the droplet 14 by breaking down the droplet 14, and causes the amplified product on the collected carrier 13 to react with the marking substance 17.

In the present embodiment, when the emulsion containing the droplets 14 and the reagent 16 for breaking down the droplets 14 are supplied to the third flow channel 130 of the specimen treatment chip 100 and the droplets 14 are broken down, the nucleic acid 10 held by the carrier 13 can be extracted from each of the droplets 14. Then, the carrier 13 extracted from the droplet 14 by breaking down the droplet 14 is collected and an amplified product on the collected carrier 13 is caused to react with a marking substance 17, so that the nucleic acid 10 extracted from each of the droplets 14 can be detected. As a result, unlike the case where the nucleic acid 10 is detected while being contained in the droplet 14, it is possible to treat a specimen in the specimen treatment chip 100 without requiring accurate control of a particle diameter of the droplet 14, generation speed thereof, and the like.

### [Configuration example of specimen treatment chip]

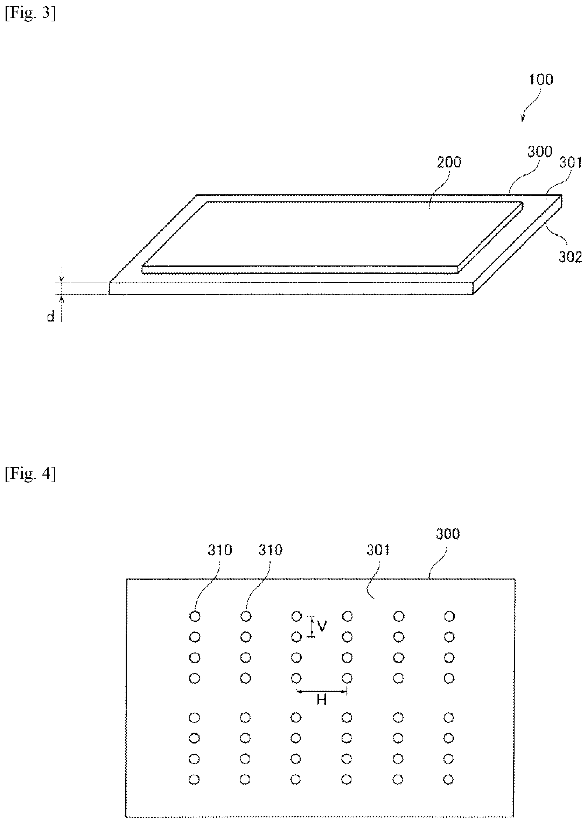

Fig. 3 illustrates a configuration example of the specimen treatment chip 100 according to the present embodiment. The specimen treatment chip 100 includes a fluid module 200 and a substrate 300. On the substrate 300, the fluid module 200 is installed. The fluid module 200 includes a first flow channel 110, a second flow channel 120, and a third flow channel 130, for example.

Fig. 4 illustrates a configuration example of the substrate 300. The substrate 300 has the shape of a flat plate, and has a first surface 301 being a main surface, and a second surface 302 (refer to Fig. 3). The second surface 302 is opposite to the first surface 301. While an upper surface of the substrate 300 serves as the first surface 301 in Fig. 3, a lower surface thereof may serve as the first surface 301. The substrate 300 is formed of a material having rigidity. For example, the substrate 300 is formed of glass. As a result, even when pressure of the liquid to be supplied to the fluid module 200 is increased according to a treatment step, sufficient pressure resistance performance can be secured for the substrate 300.

The substrate 300 has a thickness "d" (refer to Fig. 3) of 1 mm or more and 5 mm or less, for example. This enables the substrate 300 to be formed to have a sufficiently large thickness as compared with a flow channel height (on the order of 10 µm to 500 µm) of the flow channel formed in the fluid module 200. As a result, sufficient pressure resistance performance can be easily secured for the substrate 300.

The substrate 300 has a through hole 310 for injecting a liquid into the fluid module 200, for example. The through hole 310 passes through the substrate 300 in its thickness direction. The through hole 310 is connected to the flow channel of the fluid module 200, and can serve as a port 101 for supplying a liquid and a reagent into the specimen treatment chip 100 and a port 102 for recovering a liquid from the inside of the specimen treatment chip 100. As a result, the liquid can be injected through the substrate 300 that is more likely to secure pressure resistance performance than the fluid module 200 in which the flow channel is formed. This easily enables liquid injection under sufficient pressure.

Each of the port 101 and the port 102 of the specimen treatment chip 100 may be formed by a member other than the through hole 310. For example, the port 101 and the port 102 may be formed in the fluid module 200.

In the example of Fig. 4, the substrate 300 has two sets of through holes 310 of four rows by six columns. When a plurality of sets of through holes 310 is provided in the substrate 300, the fluid modules 200 for performing a series of treatment steps can be formed in columns on the substrate 300. In this case, it is possible to perform specimen treatment in parallel in one specimen treatment chip 100. The number of through holes 310 and the number of sets of through holes 310, provided in the substrate 300, are not limited to those in the example of Fig. 4. The substrate 300 may have one set of through holes 310 of eight rows by six columns.

The through holes 310 are disposed at a predetermined pitch on the substrate 300, for example. In the example of Fig. 4, each of the through holes 310 is disposed at a pitch V in the vertical direction and a pitch H in the horizontal direction. In this case, the fluid module 200 can be disposed at an arbitrary position on a pitch unit basis on the substrate 300, and its corresponding flow channel can be connected to an arbitrary through hole 310. Thus, even when structure such as a flow channel shape of the fluid module 200 is changed, the substrate 300 does not need to be changed in structure. As a result, it is possible to flexibly deal with a design change.

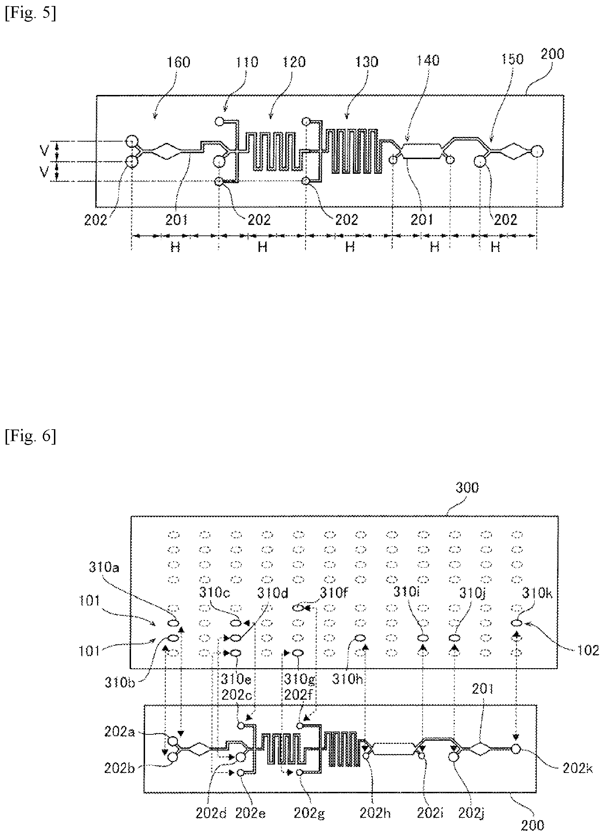

Fig. 5 illustrates a configuration example of the fluid module 200. In the present configuration example, the fluid module 200 includes a first flow channel 110, a second flow channel 120, and a third flow channel 130, and further includes a fourth flow channel 140, a fifth flow channel 150, and a sixth flow channel 160. The fluid module 200 includes channels 201 through which a liquid such as a specimen or a reagent flows, and connection portions 202 that connect to the corresponding through holes 310. Each of the connection portions 202 is used to inject a liquid into the corresponding one of the channels 201, or to suck out a liquid from the corresponding one of the channels 201. Each flow channel is composed of a combination of the corresponding one of the channels 201 and the corresponding one of the connection portions 202. Details of the fluid module 200 will be described later.

The connection portions 202 are disposed at positions corresponding to the respective through holes 310 formed at the predetermined pitches V and H on the substrate 300 to connect to the corresponding through holes 310. That is, the connection portions 202 are disposed on the fluid module 200 at pitches that are integral multiples of the respective pitches V and H of the through holes 310 of the substrate 300. The channels 201 are disposed so as to connect between the corresponding connection portions 202 disposed at predetermined pitches.

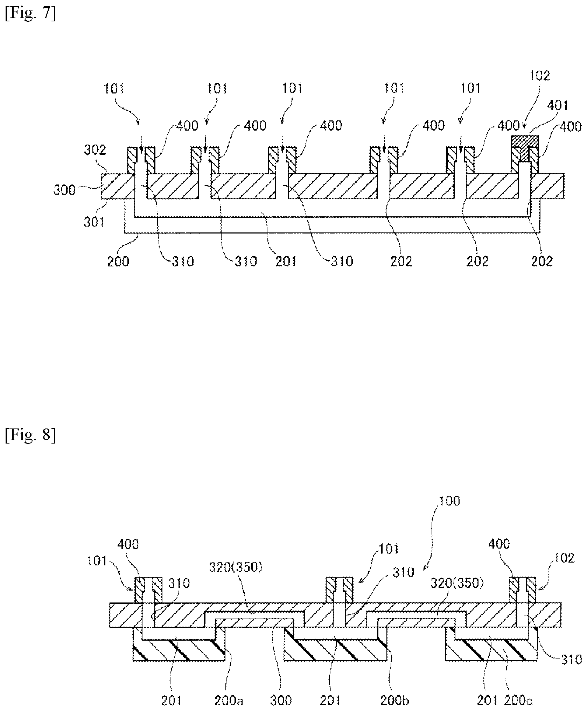

Figs. 6 and 7 each illustrate an example of placement of the fluid modules 200 on the substrate 300. In the example of Fig. 6, the connection portions 202 of the fluid module 200 are disposed so as to coincide with the corresponding through holes 310 of the substrate 300. Even when the fluid module 200 and the substrate 300, formed separately, are joined to each other, the connection portions 202 and the corresponding through holes 310 portions can be collectively connected by allowing the connection portions 202 of the fluid module 200 and the corresponding through holes 310 of the substrate 300 to coincide with each other.

The through holes 310 may be formed only at corresponding positions required for connection to various fluid modules 200 disposed on the substrate 300. In the example of Fig. 6, there are formed through holes 310a to 310k indicated by solid lines corresponding to connection portions 202a to 202k of the fluid module 200, respectively, for example. This enables structure of the substrate 300 to be further simplified. The through holes 310 may be formed at predetermined pitches on the entire substrate 300 as illustrated in Fig. 4.

The fluid module 200 is connected to the substrate 300 by solid phase bonding, for example. The solid phase bonding can use a method in which bonding surfaces are subjected to plasma treatment to form OH radicals and the bonding surfaces are bonded by hydrogen bonding, and a method such as vacuum pressure bonding, for example. The fluid module 200 and the substrate 300 can be firmly bonded by solid phase bonding. As a result, even when pressure of the liquid to be supplied to the fluid module 200 is increased, sufficient pressure resistance performance can be secured for the substrate 300. The fluid module 200 may be connected to the substrate 300 by an adhesive or the like.

The substrate 300 may include a through hole 310 for injecting an inspection liquid to be used in at least one of a plurality of steps into the specimen treatment chip 100. The through hole 310 for injecting a liquid is connected to at least one of the connection portions 202 of the fluid module 200 disposed on the substrate 300.

In the examples of Figs. 6 and 7, each of the through holes 310a to 310j of the substrate 300 serves as the port 101 for injecting a liquid. The through holes 310a to 310j are connected to the connection portions 202a to 202j of the fluid module 200, respectively. The through hole 310k is connected to the connection portion 202k of the fluid module 200 to serve as the port 102 for recovering a liquid.

The specimen and the reagent are injected into the through hole 310 serving as the port 101 with a jig such as a connector 400 (refer to Fig. 7). The jig such as the connector 400 is connected to an end portion of the through hole 310 on a side opposite to an end portion on a fluid module 200 side. That is, the jig such as the connector 400 is installed on the second surface 302 opposite to the first surface 301 of the substrate 300 on which the fluid module 200 is disposed.

The first flow channel 110 to the sixth flow channel 160 may be separately formed in the corresponding fluid modules 200. In the example of Fig. 8, the specimen treatment chip 100 includes three fluid modules 200a, 200b, and 200c, for example. In this case, the specimen treatment chip 100 is provided with a connection flow channel 350 for connecting between the corresponding fluid modules 200a to 200c to feed a liquid.

The plurality of fluid modules 200 each are separately provided on the substrate 300. That is, the plurality of fluid modules 200 are not a plurality of element parts formed in a common member, but are formed as separate parts independent from each other. Each of the fluid modules 200 has a structure in which a flow channel is formed in a block body formed of resin, glass, or the like, for example. In addition, the plurality of fluid modules 200 are installed on the substrate 300 while being separated from each other. Each of the fluid modules 200 is installed on the substrate 300 and connected through the connection flow channel 350, so that liquid can be fed between the fluid modules.

In the example of Fig. 8, the fluid modules 200a, 200b, and 200c each separately include one or more of the first flow channel 110 to the sixth flow channel 160. For example, the first flow channel 110, the second flow channel 120, and the third flow channel 130 are formed in the fluid modules 200a, 200b, and 200c, respectively.

In the present example, the substrate 300 includes substrate flow channels 320 connecting between the corresponding adjacent fluid modules 200a, 200b, and 200c. In the example of Fig. 8, the connection flow channel 350 is composed of the substrate flow channels 320 formed integrally with the substrate 300. This enables a liquid to be fed to each of the first flow channel 110 to the sixth flow channel 160 through the corresponding one of the substrate flow channels 320 in a predetermined order according to the order of the treatment steps. The connection flow channel 350 may be composed of a pipe member provided separately from the fluid modules 200 and the substrate 300, or the fluid modules 200 may be connected to each other by a combination of the substrate flow channel 320, and the pipe member or the like, for example.

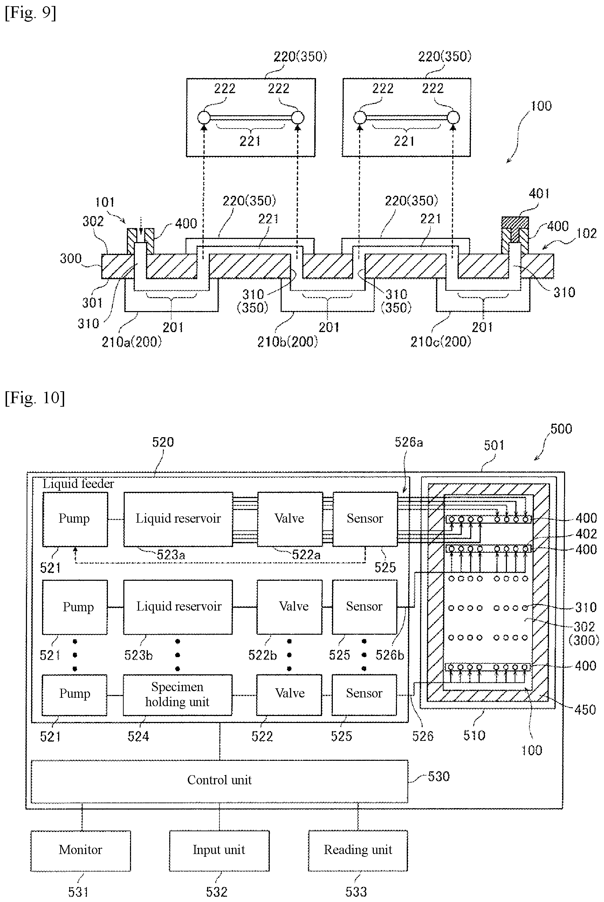

In the configuration example of Fig. 9, the specimen treatment chip 100 includes the second fluid modules 220 that are provided on the second surface 302 opposite to the first surface 301 on which the first fluid modules 210 are disposed, and that are used to connect between the corresponding first fluid modules 210a, 210b, and 210c. The substrate 300 is provided with through holes 310, and with no substrate flow channel 320. In the example of Fig. 9, the connection flow channel 350 is composed of the through hole 310 integrally formed in the substrate 300 and the second fluid module 220.

The second fluid module 220 includes connection portions 222 each for connecting to the through hole 310, and a channel 221 for connecting the connection portions 222 to each other. A liquid discharged from the first fluid module 210a is fed to the adjacent first fluid module 210b through the through hole 310 and the second fluid module 220. A liquid flowing into one of the connection portions 222 of the second fluid module 220 is discharged from the other of the connection portions 222 through the channel 221, and flows into the first fluid module 210b through the through hole. Likewise, a liquid discharged from the first fluid module 210b is fed to the adjacent first fluid module 210c through the through hole 310 and the second fluid module 220.

As a result, even a structure in which the first flow channel 110 to the sixth flow channel 160 are separately provided in the corresponding fluid modules 200 and only the through holes 310 are formed in the substrate 300 enables a liquid to be fed to each of the first flow channel 110 to the sixth flow channel 160 through the second fluid module 220, in a predetermined order according to the order of the treatment steps.

One or more of the first flow channel 110 to the sixth flow channel 160 may be formed in the second fluid module 220. In that case, one or more of the first flow channel 110 to the sixth flow channel 160 are separately formed in the corresponding one of the first fluid modules 210a, 210b and 210c, and the second fluid module 220.

Like the configuration examples of Figs. 8 and 9, when the first flow channel 110, the second flow channel 120, and the third flow channel 130 are individually formed in the corresponding fluid modules 200 and 210, the respective fluid modules 200 (210) can be different from each other in structure such as material, flow channel dimensions (width and depth), dimensions of the fluid modules 200 (210) themselves, and the like. That is, the structure of each fluid module can be optimized according to a treatment step in the corresponding one of the first flow channel 110 to the third flow channel 130.

Even in the configuration examples of Figs. 8 and 9, providing the port 101 for injecting a liquid to the fluid module 200 (210) on the substrate 300 enables a liquid to be injected through the substrate 300 that is more likely to secure pressure resistance performance than the fluid module 200 in which the flow channel is formed. This easily enables liquid injection under sufficient pressure.

### [Configuration example of specimen treatment apparatus]

Fig. 10 illustrates a configuration example of the specimen treatment apparatus 500. The specimen treatment apparatus 500 has functions such as liquid injection into the specimen treatment chip 100, liquid recovery from the specimen treatment chip 100, detection of a reaction occurring in the specimen treatment chip 100, and the like.

In the configuration example of Fig. 10, a liquid feeder 520 includes a pump 521 that controls pressure for driving a liquid, and a valve 522 that opens and closes a supply path of pressure to the liquid. The liquid feeder 520 further includes a liquid reservoir 523 for containing liquid to be injected into the specimen treatment chip 100, and a specimen holding unit 524. In addition, the liquid feeder 520 includes a flow rate sensor 525 for measuring a flow rate of liquid flowing in the specimen treatment chip.

The pump 521, the liquid reservoir 523, the valve 522, and the flow rate sensor 525 are connected in order by a liquid feeding pipe 526. The specimen treatment apparatus 500 injects liquid into the specimen treatment chip 100 and recovers liquid from the specimen treatment chip 100 through the connector 400 by using the pump 521, the liquid reservoir 523, and the valve 522. In the example of Fig. 10, one set of the pump 521, the liquid reservoir 523, and the valve 522 corresponds to a predetermined connector 400. For example, the specimen treatment apparatus 500 has the same number of sets of the pump 521, the liquid reservoir 523, and the valve 522 as the number of the connectors 400 connectable to the specimen treatment chip 100 (or the number of rows of ports). However, at least one liquid reservoir 523 is configured as the specimen holding unit 524 that holds a specimen.

For example, a plurality of liquid reservoirs 523 and a plurality of valves 522 may be connected to one pump 521. The valve 522 switches a route to enable a plurality of kinds of liquid and reagent to be supplied to the specimen treatment chip 100 by the common pump 521.

The pump 521 applies pressure to the liquid reservoir 523 and the specimen holding unit 524. When the pump 521 applies positive pressure to the liquid reservoir 523, liquid is fed from the liquid reservoir 523. When the pump 521 applies negative pressure to the liquid reservoir 523, liquid flows into the liquid reservoir 523 from the specimen treatment chip 100. The pump 521 is a pressure pump that supplies air pressure, for example. Besides this, a syringe pump, a diaphragm pump, or the like can be used as the pump 521.

The control unit 530 can individually control operation of each pump 521. The control unit 530 individually controls each pump 521 to enable individual control of feeding a liquid for each of the flow channels disposed in the specimen treatment chip 100.

For example, the control unit 530 controls the liquid feeder 520 so as to cause a liquid containing a nucleic acid 10 to continuously flow into the first flow channel 110, the second flow channel 120, and the third flow channel 130. As a result, as compared with a case of performing intermittent or discontinuous liquid feeding in which a liquid is stopped to flow in the course of flowing through from the first flow channel 110 to the third flow channel 130, allowing a liquid to continuously flow from the first flow channel 110 to the third flow channel 130 enables time required for specimen treatment to be easily shortened.

In the configuration of Fig. 10, the flow rate sensor 525 detects a flow rate (e.g., a unit is µL/min) of a liquid flowing through the liquid feeding pipe 526. The flow rate sensor 525 feeds back a detection result of the flow rate to the pump 521. The pump 521 controls pressure in response to feedback from the flow rate sensor 525.

The flow rate sensor 525 may transmit feedback to the control unit 530. The control unit 530 controls pressure of the liquid feeder 520 for feeding liquid, in accordance with a flow rate measured by the flow rate sensor 525. This makes it possible to accurately control supply pressure when a specimen containing a nucleic acid or a reagent is supplied to the specimen treatment chip 100.

The connector 400 is provided in a lid 621 described later of the installation unit 510, for example. The connector 400 is connected to the liquid feeding pipe 526. In the connector 400, liquid such as a specimen is fed to the specimen treatment chip 100 through the connector 400. In addition, liquid is recovered from the specimen treatment chip 100 through the connector 400.

The specimen treatment chip 100 is set in the installation unit 510. For example, the specimen treatment chip 100 is held such that the second surface 302 of the substrate 300 faces upward, and the through hole 310 is connected at its end portion on a second surface 302 side to the connector 400.

The specimen treatment chip 100 may include a fixture 450 for installation in the installation unit 510. The fixture 450 may be detachable from the installation unit 510, or may be fixed to the installation unit 510.

In addition, the specimen treatment apparatus 500 can include a monitor 531, an input unit 532, a reading unit 533, and the like. The control unit 530 causes the monitor 531 to display a predetermined display screen corresponding to operation of the specimen treatment apparatus 500. The specimen treatment apparatus 500 may be connected to an external computer (not illustrated) to display a screen in a monitor of the computer. The input unit 532 is composed of a keyboard and the like, for example, and has a function of receiving information input. The reading unit 533 is composed of a code reader for a bar code, a two-dimensional code, or the like, and a tag reader for an RFID tag, or the like, and has a function of reading out information given to the specimen treatment chip 100. The reading unit 533 can also read out information such as a specimen container (not illustrated) for containing a specimen.

### (Configuration example of valve)

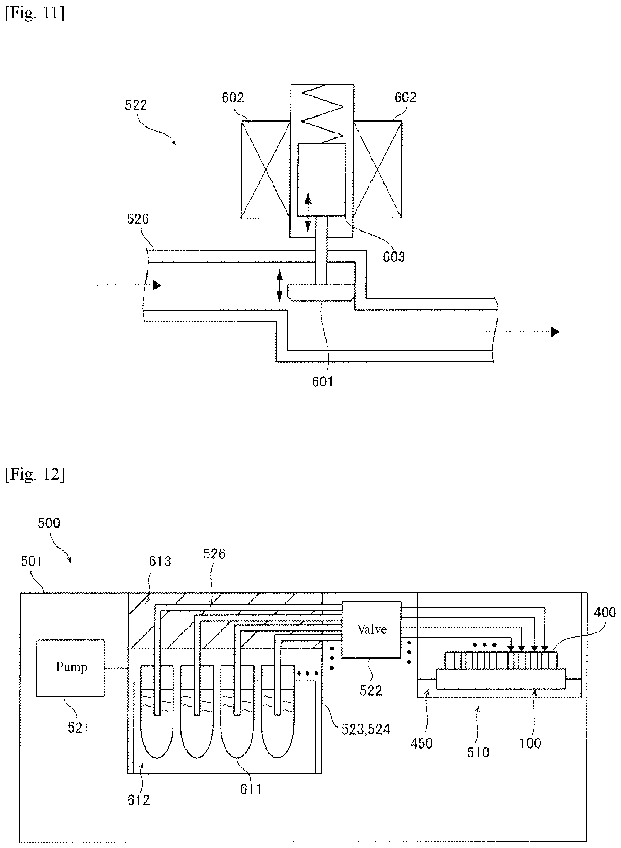

Fig. 11 illustrates a configuration example of the valve 522. The valve 522 controls an outflow of liquid from the liquid reservoir 523 and an inflow of the liquid into the liquid reservoir 523 by using a valve 601.

The valve 522 is an electromagnetic valve, for example. The valve 522 includes a coil 602. The coil 602 moves a plunger 603 between an open position and a closed position by using a magnetic field generated by electric current flowing through the coil 602. The control unit 530 controls electric current flowing through the coil 602. The valve 601 opens and closes the liquid feeding pipe 526 in accordance with a movement of the plunger 603.

As in the example of Fig. 10, a plurality of valves 522 is disposed in the specimen treatment apparatus 500. The control unit 530 is capable of individually controlling opening and closing of each valve 522.

The control unit 530 controls opening and closing of the respective valves 522 of the liquid feeder 520 to feed a liquid in the specimen treatment chip 100 to the first flow channel 110, the second flow channel 120, and the third flow channel 130 by using pressure. As a result, a plurality of kinds of liquid and reagent can be easily and efficiently supplied to each of the first flow channel 110, the second flow channel 120, and the third flow channel 130, of the specimen treatment chip 100, at desired timing by merely controlling opening and closing timing of each of the valves 522.

The control unit 530 controls timing of opening of the valve 522 on the basis of an elapsed time from an injection of liquid into the specimen treatment chip 100, or the amount of an injection of the liquid into the specimen treatment chip 100, for example. This makes it possible to accurately control the amount of supply of liquid into the specimen treatment chip 100 on the basis of an elapsed time under a constant flow rate and the injection amount of the liquid. As a result, quantitative supply of various kinds of liquid suitable for each channel of the specimen treatment chip 100 becomes possible. The control unit 530 may determine timing of opening of each valve 522 on the basis of a result of image analysis of a flow of the liquid in the specimen treatment chip 100, for example.

### (Configuration example of liquid feeding pipe)

For example, the specimen treatment apparatus 500 includes the number of liquid feeding pipes 526a corresponding to the number of holes 402 of the connector 400, as illustrated between a liquid reservoir 523a and a valve 522a as well as between the valve 522a and the connector 400. In the example of Fig. 10, eight liquid feeding pipes 526a are disposed between the liquid reservoir 523a and the valve 522a as well as between the valve 522a and the connector 400. In this case, the valve 522a is disposed for each of the eight liquid feeding pipes 526a.

For example, the specimen treatment apparatus 500 may include a liquid feeding pipe 526b branching to the holes 402 of the connector 400, as illustrated between the liquid reservoir 523b and the valve 522b as well as between the valve 522b and the connector 400. In the example of Fig. 10, one liquid feeding pipe 526b is disposed between the liquid reservoir 523b and the valve 522b, and the liquid feeding pipe 526b branches to each of the holes 402 of the connector 400.

### (Configuration example of liquid reservoir and specimen holding unit)

Fig. 12 illustrates a configuration example of the liquid reservoir 523 and the specimen holding unit 524.

Liquid containers 611 each such as for a specimen and a reagent are disposed in a container installation unit 612 of the liquid reservoir 523 or the specimen holding unit 524. As illustrated in Fig. 12, a plurality of the container installation units 612 may be disposed, or the single container installation unit 612 may be disposed.

The liquid reservoir 523 and the specimen holding unit 524 are hermetically sealed by a lid 613. The lid 613 is provided with the liquid feeding pipes 526. When the liquid reservoir 523 is sealed with the lid 613, the liquid feeding pipes 526 are inserted into the corresponding containers 611 for a specimen or a reagent. The liquid feeding pipes 526 provided in the lid 613 are connected to the specimen treatment chip 100 through the valve 522. The pump 521 adjusts pressure in the liquid reservoir 523 sealed with the lid 613. When the pressure in the liquid reservoir 523 is increased to open the valve 522, liquid in each of the containers 611 is supplied to a specimen treatment chip 100 side.

The control unit 530 determines a liquid reservoir 523 in which a liquid is to be contained and a kind of liquid to be contained in the liquid reservoir 523, for example, and notifies the determined liquid reservoir 523 and the kind of liquid to be contained. For example, the notification can be achieved by a method such as for displaying the liquid reservoir 523 in which a liquid is to be contained, and the kind of liquid to be contained in the liquid reservoir 523, in the monitor 531 of the specimen treatment apparatus 500 or a monitor (not illustrated) of a computer connected to the specimen treatment apparatus 500. This enables erroneous operation by a user to be prevented.

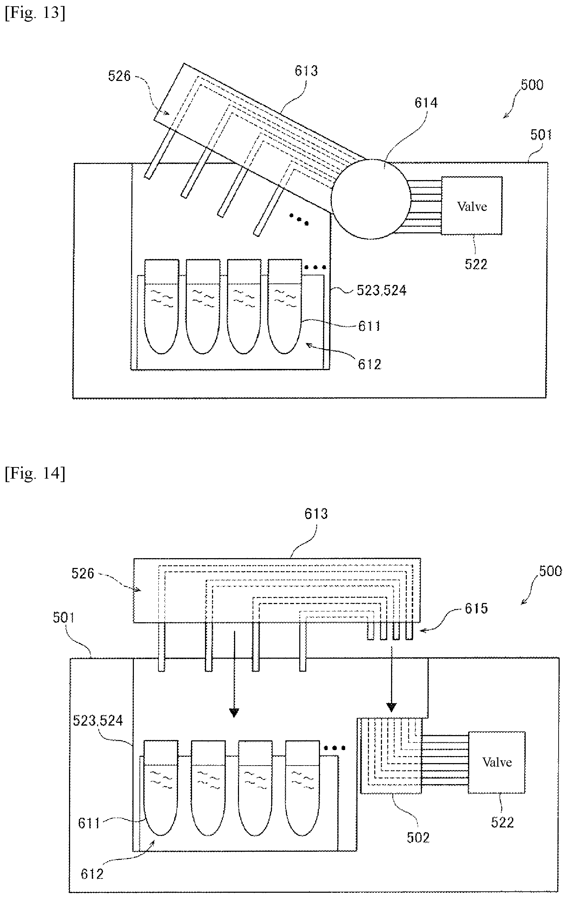

Figs. 13 and 14 each illustrate a configuration example of the lid 613 for a liquid reservoir.

The lid 613 illustrated in Fig. 13 is connected to a specimen treatment apparatus main body 501 with a hinge 614. The lid 613 is moved by rotation of the hinge 614 to be able to open and close the inside of the liquid reservoir 523 or the specimen holding unit 524. At least some of the liquid feeding pipes 526 provided in the lid 613 are each composed of a rubber tube or the like to be deformable in response to opening and closing of the lid 613.

The lid 613 illustrated in Fig. 14 is detachable from the specimen treatment apparatus main body 501. When the lid 613 is attached to the specimen treatment apparatus main body 501, the connector 615 of the lid 613 and the connector 502 on a specimen treatment apparatus 500 side are connected to each other, and then the liquid feeding pipes 526 between the lid 613 and the valve 522 are connected.

The lid 613 is detachable from the specimen treatment apparatus 500, maintenance of the liquid feeding pipe 526 can be performed only by replacing the lid 613 when the liquid feeding pipe 526 is deteriorated due to contamination or the like.

### (Configuration example of lid of installation unit)

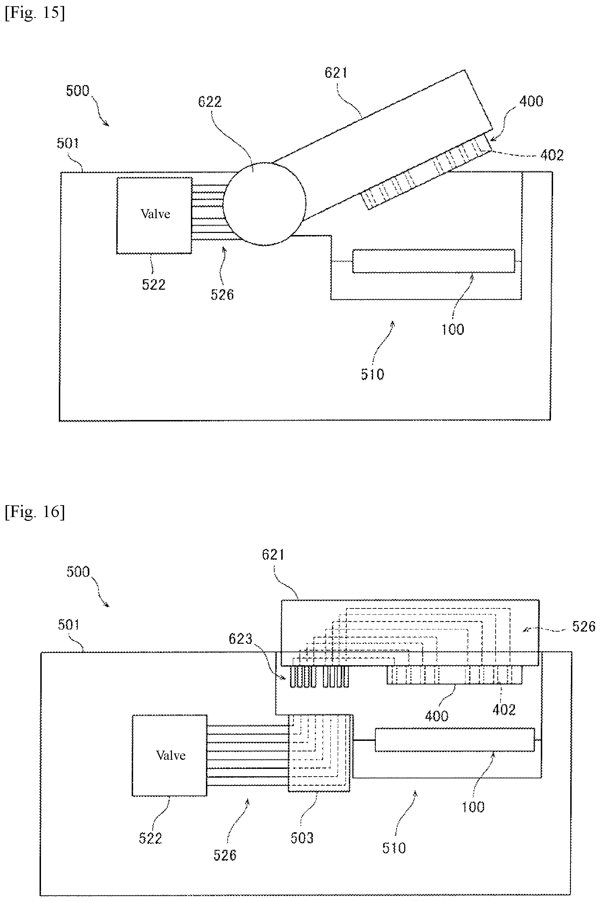

The installation unit 510 may be provided with a lid 621 corresponding to the installation unit 510. Figs. 15 and 16 each illustrate a configuration example of the lid 621 of the installation unit 510. The lid 621 is provided so as to cover the specimen treatment chip 100 set in the installation unit 510.

The lid 621 illustrated in Fig. 15 is connected to a specimen treatment apparatus main body 501 with a hinge 622. The lid 621 is opened and closed by rotation of the hinge 622. At least some of the liquid feeding pipes 526 provided in the lid 621 are each composed of a rubber tube or the like to be deformable in response to opening and closing of the lid 621.

The lid 621 may include a connector 400 for supplying or recovering a liquid through a port provided at a predetermined position on the specimen treatment chip 100. The port is a through hole 310 serving as a port 101 for injecting a liquid or a reagent, or a through hole 310 serving as a port 102 for recovering a liquid, for example. The tip of each of the liquid feeding pipes 526 extending from the valve 522 is connected to the hole 402 of the connector 400. Liquid is fed between the specimen treatment chip 100 and the liquid feeding pipe 526 through the connector 400. This makes it possible to connect the specimen treatment chip 100 installed in the installation unit 510 and the connector 400 to each other only by closing the lid 621 of the installation unit 510.

The lid 621 illustrated in Fig. 16 is detachable from the specimen treatment apparatus main body 501.

When the lid 621 is attached to the specimen treatment apparatus main body 501, the connector 623 of the lid 621 and the connector 503 of the specimen treatment apparatus 500 are connected to each other, and then the liquid feeding pipes 526 between the lid 621 and the valve 522 are connected. In addition, the connector 400 of the lid 621 is connected to the port of the specimen treatment chip 100. Liquid is fed between the specimen treatment chip 100 and the liquid feeding pipe 526 through the connectors 503, 623, and 400.

When the lid 621 is configured to be detachable from the specimen treatment apparatus main body 501 as described above, maintenance of the liquid feeding pipe 526 can be performed only by replacing the lid 621 when the liquid feeding pipe 526 is deteriorated due to contamination or the like.

### (Configuration Example of Connector)

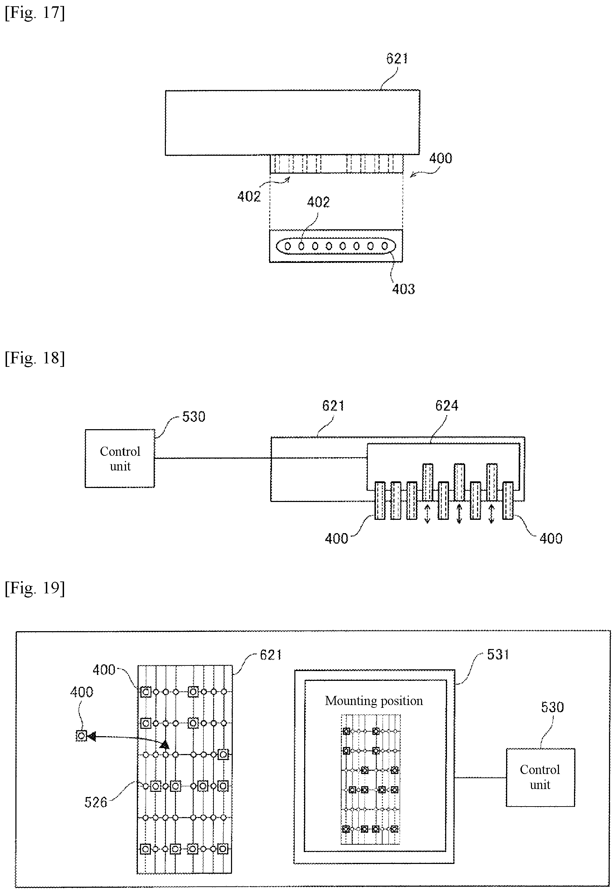

Figs. 17 to 19 each illustrate a configuration example of the connector 400.

The connector 400 is provided on the lid 621. The connector 400 has the hole 402 for connecting to the through hole 310 of the substrate 300. The connector 400 is installed at a position corresponding to the through hole 310 of the substrate 300. The connector 400 may be provided only at a position corresponding to an arbitrary through hole 310.

Liquid, such as a specimen and a reagent, is injected into the specimen treatment chip 100 from the liquid feeding pipe 526 through the hole 402. The liquid flowing through the specimen treatment chip 100 is recovered from the specimen treatment chip 100 through the hole 402. An arbitrary through hole 310 can be sealed by inserting the plug 401 (refer to Fig. 7, etc.) into the corresponding hole 402.

The connector 400 is provided with a sealing material such as a gasket 403 on its contact surface with the specimen treatment chip 100. The gasket 403 prevents liquid leakage and foreign matter contamination between the ports 101 and 102, and the holes 402.

The through hole 310 through which a liquid is injected or recovered by the connector 400 varies according to a shape of the flow channel disposed in the specimen treatment chip 100. Thus, the connector 400 does not need to be disposed in all the through holes 310.

For example, the lid 621 may be capable of accommodating the connector 400 inside the lid 621. In the example of Fig. 18, the lid 621 includes a plurality of connectors 400, and a driving unit 624 for moving each of the plurality of connectors 400 inward and outward of the lid 621. Then, the control unit 530 determines the corresponding one of the connectors 400 to be accommodated inside the lid 621 on the basis of port position of the specimen treatment chip 100, and instructs the lid 621 to accommodate the determined connector 400. When the connector 400 specified by the control unit 530 protrudes to the outside of the lid 621, the driving unit 624 moves the connector 400 backward to the inside of the lid 621.

According to the present configuration, only the connector 400 necessary for use of the specimen treatment chip 100 can be automatically connected to the specimen treatment chip 100. In addition, it is possible to prevent the connector 400 from being installed in a wrong position.

The connector 400 may be configured to be detachable from the lid 621. In the example of Fig. 19 illustrating a lower surface of the lid 621, the lid 621 is configured such that a plurality of connectors 400 is detachable. A user of the specimen treatment apparatus 500 can mount a necessary connector 400 at a predetermined position of the lid 621 according to port position of specimen treatment chip 100. In this case, the control unit 530 notifies a position where the corresponding one of the connectors 400 is to be mounted on the basis of the port position of the specimen treatment chip 100, for example. The notification can be achieved by using a method such as for displaying a position where the connector 400 is to be mounted in the monitor 531 of the specimen treatment apparatus 500, or in a monitor (not illustrated) of a computer connected to the specimen treatment apparatus 500. This enables only the connector 400 necessary for use of the specimen treatment chip 100 to be connected to the specimen treatment chip 100 with a simple configuration, so that wrong attachment of the connector 400 by a user can be prevented.

### <Configuration Example of Fixture>

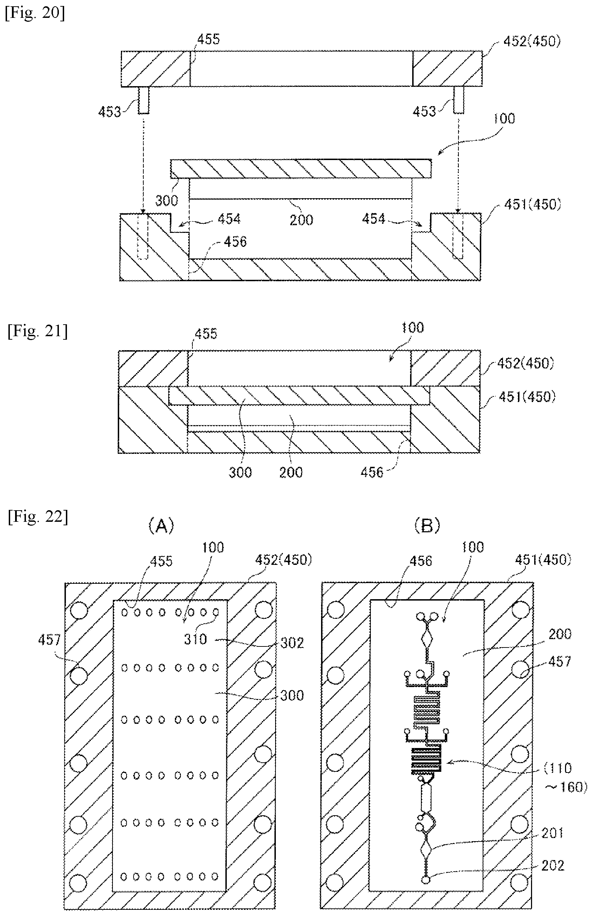

Figs. 20 to 22 each illustrate an example of the fixture 450 used for installing the specimen treatment chip 100 in the specimen treatment apparatus 500.

As illustrated in Fig. 20, the specimen treatment chip 100 is fixed with fixtures 451 and 452, for example. The fixtures 451 and 452 are fixed with fitting members 453. For example, the specimen treatment chip 100 is horizontally positioned by a positioning portion 454 formed in the fixture 451 on a lower side. In the example of Fig. 20, the positioning portion 454 is composed of a stepped portion that is recessed. The positioning portion 454 determines a relative position between the specimen treatment chip 100 and the fixtures 451 and 452.

Fig. 21 is a side view of the specimen treatment chip 100 in a state of being fixed with the fixtures 451 and 452. The specimen treatment chip 100 composed of a substrate 300 provided with a bonded fluid modules 200 is fixed with the fixtures as illustrated in Fig. 21.

As illustrated in Fig. 22 (A), the fixture 452 has an opening 455 formed of a through hole at a position corresponding to the substrate 300. The connector 400 and the like of the specimen treatment apparatus 500 can be connected to the substrate 300 from above through the opening 455. In addition, as illustrated in Fig. 22 (B), the fixture 451 has an opening portion 456 formed of a through hole at a position corresponding to the substrate 300 and the fluid module 200, so that the substrate 300 and the fluid module 200 can be connected from below through the opening portion 456.

When the specimen treatment chip 100 held by the fixtures 451 and 452 is installed in the installation unit 510, or when the specimen treatment chip 100 is set to the fixture 451 fixed to the installation unit 510 and the fixture 452 is attached to the fixture 451, the specimen treatment chip 100 is set in the installation unit 510. The fixture 452 may be fixed to the lid 621 of the installation unit 510 so that the fixture 452 is attached to the fixture 451 at the same time when the lid 621 is installed.

As illustrated in Fig. 22, the fixtures 451 and 452 each may have attachment holes 457 for disposing various treatment units provided in the specimen treatment apparatus 500. In the example of Fig. 22, a plurality of the attachment holes 457 is provided outside the opening 455 along a long side of the fixture 452 (451).

### (Installation example of various units)

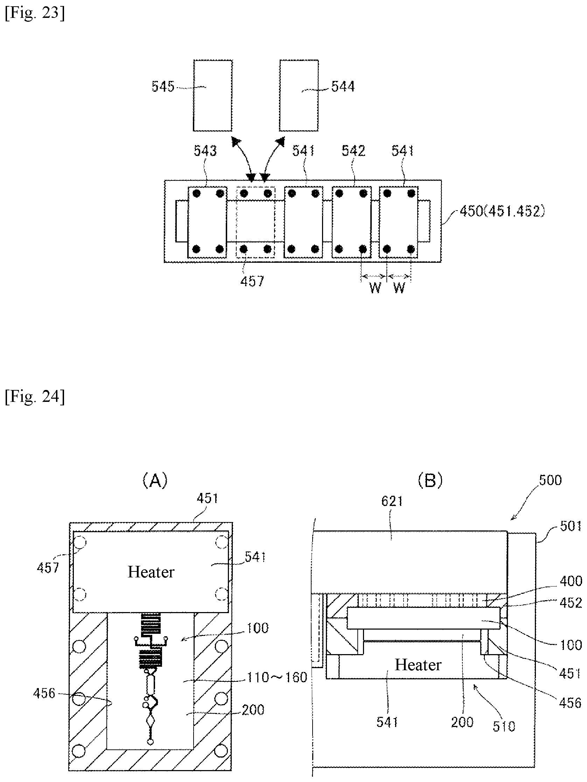

Fig. 23 illustrates an installation example of treatment units used for various treatment steps of the specimen treatment apparatus 500.

As illustrated in Figs. 23, a heater unit (heater 541) for heating a liquid in the fluid module 200, a magnet unit 542 for applying a magnetic force to the liquid in the fluid module 200, a cooling unit 543 for cooling the liquid in the fluid module 200, a detection unit 544 for detecting an object component in the specimen treatment chip 100, a camera unit 545 for photographing a flow of the liquid in the fluid module 200, and the like are attached to the fixture 451 or 452 using the attachment holes 457, for example. The connector 400 may be attached to the fixture 451 or 452. The treatment unit may be a complex type unit having a plurality of functions of the above functions. For example, a treatment unit having a function of heating a liquid and a function of applying magnetic force to a liquid may be used.

When these treatment units and the specimen treatment chip 100 are simply attached to the fixtures 451 and 452, relative positioning between each of the treatment units and the specimen treatment chip 100 can be easily performed with the fixture 451 (452).

For example, a plurality of the attachment holes 457 is provided at a predetermined pitch W. Thus, even when specimen treatment chips 100 different in placement or shape of a flow channel formed in the fluid module 200 are used, a position of each of the treatment units can be freely changed in units of the pitch W according to flow channel structure. The pitch W may be equal to the pitch H of the through hole 310 of the substrate 300, or may be an integral multiple of the pitch H, for example. In this case, it is possible to easily allow a position of each flow channel of the fluid modules 200 and a position of the corresponding one of the treatment units to coincide with each other.

### <Heater Unit>

Fig. 24 illustrates a placement example of the heater 541 in the specimen treatment apparatus 500.

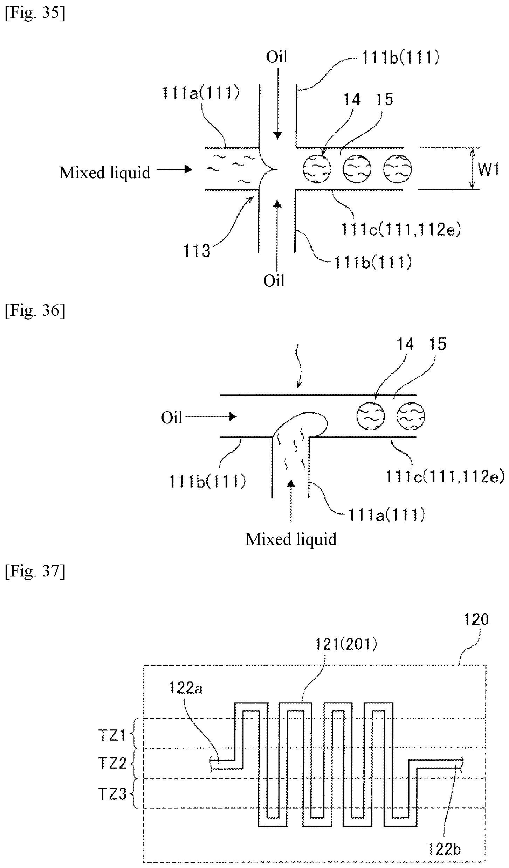

The heater 541 adjusts temperature of the specimen treatment chip 100. For example, the heater 541 heats the specimen treatment chip 100 to amplify DNA in the fluid module 200 by PCR. More specifically, the heater 541 forms a plurality of temperature zones TZ1, TZ2, and TZ3 (refer to Fig. 37) in the second flow channel 120 of the specimen treatment chip 100. When the heater 541 forms the plurality of temperature zones TZ1, TZ2, and TZ3, thermal cycle processing can be performed merely by allowing a liquid flowing through the second flow channel 120 to pass through the respective temperature zones TZ1 to TZ3. In this case, each of the temperature zones TZ1 to TZ3 only needs to be maintained at a different constant temperature, so that temperature control can be facilitated as compared with a case where the thermal cycle process is performed by periodically changing temperature of the entire heater 541.

The heater 541 is provided in the installation unit 510. For example, the heater 541 is attached to the fixture 451 on a lower surface side of the specimen treatment chip 100. The heater 541 adjusts temperature of the specimen treatment chip 100 from the lower surface side of the specimen treatment chip 100 installed in the installation unit 510. The heater 541 is disposed at a position corresponding to the flow channel to be controlled for temperature.

The heater 541 may be movable. The control unit 530 of the specimen treatment apparatus 500 causes the heater 541 to be moved such that the heater 541 is disposed at a position corresponding to a position of the flow channel to be controlled for temperature in the fluid module 200 mounted on the specimen treatment chip 100.

### <Detection Unit>

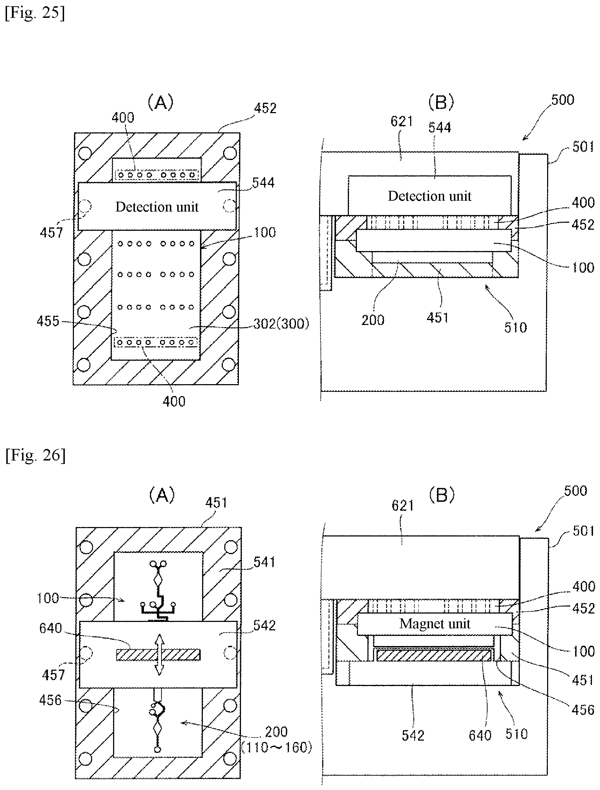

Fig. 25 illustrates a configuration example of the detection unit 544 of the specimen treatment apparatus 500.

The detection unit 544 detects fluorescence of a marking substance binding to a nucleic acid, for example. The detection unit 544 is a photomultiplier, for example. The detection unit 544 is attached to the fixture 452 on an upper surface side of the specimen treatment chip 100, for example. The detection unit 544 may be provided in the lid 621. The detection unit 544 detects fluorescence from between the connectors 400 connected to the specimen treatment chip 100. The detection unit 544 may be provided in the fixture 451 on the lower surface side of the specimen treatment chip 100, or in the specimen treatment apparatus main body 501. In this case, the detection unit 544 detects fluorescence from the lower surface side of the specimen treatment chip 100.

### <Magnet Unit>

Fig. 26 illustrates a configuration example of the magnet unit 542 used for controlling magnetic particles contained in a liquid in the specimen treatment chip 100. When using a magnetic particle as a carrier 13, the magnet unit 542 performs treatment for collecting the carrier 13 by applying a magnetic force to the magnetic particle. As a result, even in a minute flow channel and well provided in the specimen treatment chip 100, the carrier 13 in the liquid can be easily collected by the magnetic force.

The magnet unit 542 is attached to the fixture 451 on the lower surface side of the specimen treatment chip 100, for example. The magnet unit 542 may be provided in the specimen treatment apparatus main body 501. The magnet unit 542 includes a magnet 640. The magnet 640 applies a magnetic force to magnetic particles contained in liquid in the specimen treatment chip 100. For example, the magnet 640 fixes the magnetic particle at a predetermined position within the flow channel of the fluid module 200 by using a magnetic force. The magnetic particles are cleaned by causing a cleaning liquid to flow to the magnetic particles fixed at a predetermined position. For example, the magnet unit 542 allows the magnet 640 to be movable in a longitudinal direction of the specimen treatment chip 100.

While illustration is eliminated, the same applies to the camera unit 545 and the cooling unit 543.

### (Operation of specimen treatment apparatus)

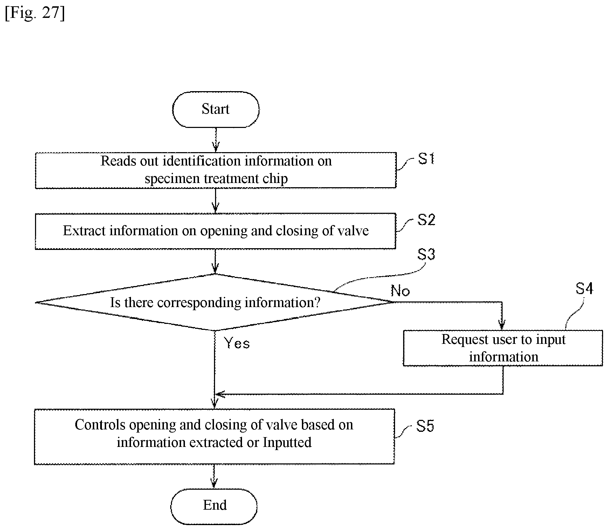

With reference to flowcharts of Figs. 27 to 29, an example of operation of the specimen treatment apparatus 500 will be described.

### <Control of opening and closing of valve>

In step S1 of Fig. 27, the specimen treatment apparatus 500 reads out identification information given to the specimen treatment chip 100. The identification information is given in the form of a bar code or QR code (registered trademark), for example, and the specimen treatment apparatus 500 reads out the identification information with the reading unit 533. The read-out information is transmitted to the control unit 530.

The identification information includes information determined according to structure of the flow channel, such as a combination of the first flow channel 110 to the sixth flow channel 160 of the specimen treatment chip 100, and placement of the connection portions 202, for example. The identification information may include information on other elements (e.g., a kind of assay method and the like) along with the structure of the flow channel of the specimen treatment chip 100. The identification information may include the following information, for example.
- ID of the through hole 310 into which a liquid is to be injected and positional information thereon.
- ID of the through hole 310 from which a liquid is to be recovered and positional information thereon.
- Information indicating the order of injecting or recovering of liquid.
   (e.g., the order is expressed by the placement order of the ID of the through hole 310 described above)
- Information indicating timing of injecting or recovering of liquid.
   (e.g., the timing is expressed by an elapsed time from start of injection of a liquid or the amount of the injection, and the timing is set for each ID of the through hole 310 to which the liquid is to be injected)
- ID of liquid (a reagent, etc.) used for inspection.
- Information indicating a position where liquid used for inspection is stored.
   (e.g., the storage position is expressed by a number indicating the liquid reservoir 523 to which the liquid is to be stored, or the like.)

In step S2, the control unit 530 extracts information on opening and closing of a valve from read-out identification information. For example, the control unit 530 extracts ID of the through hole 310 related to injection or recovery of a liquid and positional information thereon.

In step S3, the control unit 530 determines whether there is corresponding information. When it is determined that information on opening and closing of the valve is not included in the identification information, the control unit 530 causes processing to proceed to step S4. In this case, in step S4, the control unit 530 causes the monitor 531 of the specimen treatment apparatus 500 or a monitor (not illustrated) of a computer connected to the specimen treatment apparatus 500 to display contents prompting input of information on opening and closing of the valve.

When it is determined in step S3 that the identification information includes information on opening and closing of the valve, the control unit 530 causes the processing to proceed to step S5. In step S5, the control unit 530 controls opening and closing of each of the valves 522 of the liquid feeder 520 on the basis of the identification information read out from the specimen treatment chip 100 by the reading unit 533. When receiving information on opening and closing of the valve through the input unit 532, the control unit 530 controls the opening and closing of each of the valves 522 of the liquid feeder 520 on the basis of the received identification information.

The control unit 530 controls opening and closing of the valve 522 corresponding to the position of the through hole 310 related to injection or recovery of liquid. The control unit 530 controls the valve 522, corresponding to the position of the through hole 310 not related to the injection or recovery of the liquid, so as to be always closed during inspection.

When the control unit 530 is configured so as to control opening and closing of the valve 522 on the basis of identification information indicating structure of the flow channel of the fluid module 200, as described above, a user does not need to individually designate the valve 522 to be controlled for opening and closing every time using the specimen treatment chip 100 even in a case where the through hole 310 for injecting or recovering liquid differs according to the structure of the flow channel of the fluid module 200.

In addition, when the control unit 530 is configured so as to control opening and closing of the valve 522 on the basis of the identification information received from the input unit 532, the valve 522 to be controlled for opening and closing can be determined by a user who needs to only input the identification information at the time when the specimen treatment chip 100 is used.

Further, when the control unit 530 is configured so as to control opening and closing of the valve 522 on the basis of the identification information read out from the specimen treatment chip 100 by the reading unit 533, the identification information does not need to be input when the specimen treatment chip 100 is used. As a result, preparation work related to opening and closing of the valve 522 becomes unnecessary, so that convenience of the specimen treatment apparatus 500 is improved.

### <Control of timing of opening and closing of valve>

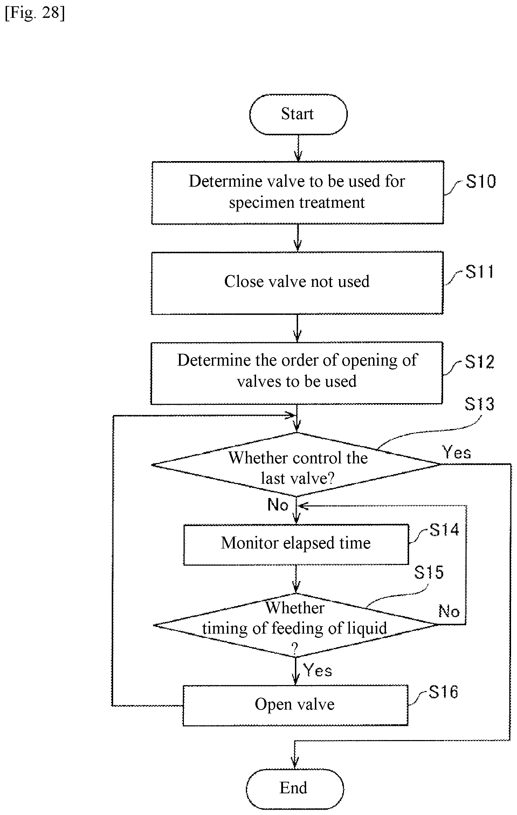

Fig. 28 illustrates an operation example when the control unit 530 controls timing of opening of the valve 522.

In step S10, the control unit 530 determines the valve 522 to be used for specimen treatment on the basis of structure of the flow channel of the fluid module 200. The control unit 530 determines a position of the port 101 provided on the specimen treatment chip 100 to inject a liquid into the fluid module 200 on the basis of structure of the flow channel of the fluid module 200, according to the operation illustrated in Fig. 27, for example. That is, the control unit 530 determines the through hole 310 serving as the port 101 for injecting the liquid. The control unit 530 controls opening and closing of each valve 522 of the liquid feeder 520 on the basis of the determined position of the port 101.

In step S11, the control unit 530 closes the valve 522 that is not used. In step S12, the control unit 530 determines the order of opening of the valves 522 used for specimen treatment. For example, the control unit 530 determines the order of opening of the valves 522 on the basis of information (information indicating the order of injecting or recovering of liquid) included in the above-described identification information.

In step S13, the control unit 530 determines whether control of the last valve 522 in the determined order is completed. When it is determined that the control of the last valve 522 is not completed, the control unit 530 monitors an elapsed time from the start of injection of liquid into the specimen treatment chip 100 in step S14. For example, the control unit 530 monitors an elapsed time from the moment when the first valve 522 is opened.

In step S15, the control unit 530 determines whether timing of feeding of liquid into the specimen treatment chip 100 arrives. When it is determined that the timing of feeding of liquid into the specimen treatment chip 100 arrives, the control unit 530 opens the corresponding valve 522 in step S16. For example, the control unit 530 determines the timing of feeding of liquid based on whether the above-described elapsed time reaches timing extracted from the identification information. When it is determined that the elapsed time does not reach the timing of feeding of liquid, the control unit 530 causes processing to return to step S14 to monitor the elapsed time.

The control unit 530 repeats the operation of steps S14 to S16 until having performed the operation for all the valves 522 determined to be used in the specimen treatment. When completing control of the last valve 522, the control unit 530 ends the processing.

### <Storage process of liquid into liquid reservoir>

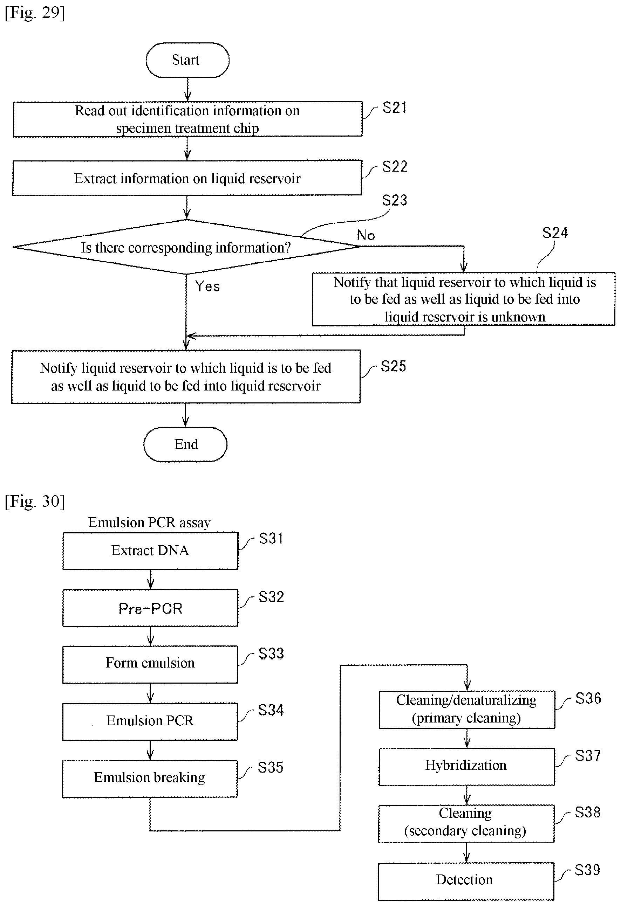

Fig. 29 illustrates an example of operation when liquid to be used for inspection is stored in a liquid reservoir.

Step S21 includes the same operation as that in step S1 in Fig. 27.

In step S22, the control unit 530 extracts information on the liquid reservoir 523 from the read-out identification information. For example, the control unit 530 extracts information indicating liquid (a reagent or the like) used for inspection, and information indicating a position storing the liquid to be used for the inspection.

In step S23, the control unit 530 determines whether there is corresponding information. When it is determined that information on the liquid reservoir 523 is not included in the identification information, the control unit 530 causes the monitor 531 to display the fact that the liquid reservoir 523 to which liquid is to be fed as well as the liquid to be fed into the liquid reservoir 523 is unknown, in step S24. The display may be performed by a monitor (not illustrated) of a computer connected to the specimen treatment apparatus 500.

When it is determined that related information is included in the identification information, the control unit 530 causes the monitor 531 to display the liquid reservoir 523 to which liquid is to be fed as well as a kind of liquid to be fed into the liquid reservoir 523, on the basis of the extracted information, in step S25. The liquid reservoir 523 as well as the kind of liquid is displayed to prevent erroneous operation by a user. The display may be performed by a monitor (not illustrated) of a computer connected to the specimen treatment apparatus 500.

### [Configuration example of specimen treatment chip]

Next, a specific configuration example of the specimen treatment chip 100 will be described. An example of performing an emulsion PCR assay using the above-described specimen treatment chip 100 will be described.

### <Description of emulsion PCR assay>

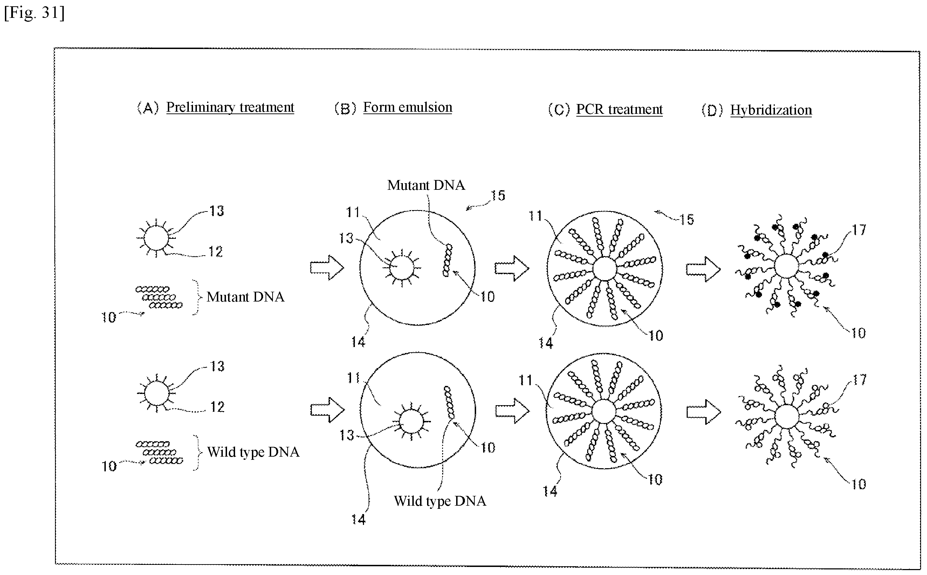

Fig. 30 illustrates an example of a flow of an emulsion PCR assay. Fig. 31 is a diagram for illustrating progress of reaction in the emulsion PCR assay. Here, it is assumed that the nucleic acid 10 is DNA and the carrier 13 is a magnetic particle.

In step S31, DNA is extracted from a sample such as blood by pretreatment (refer to Fig. 31(A)). The pretreatment may be performed using a dedicated nucleic acid extraction device, or a pretreatment mechanism may be provided in the specimen treatment device 500.

In step S32, the extracted DNA is amplified by Pre-PCR treatment (refer to Fig. 31(A)). The Pre-PCR treatment is performed to preliminarily amplify the DNA contained in an extraction liquid after the pretreatment to the extent that subsequent emulsion creation treatment is possible. In the Pre-PCR treatment, the extracted DNA, and a reagent for PCR amplification, containing polymerase and primer, are mixed, and the DNA in the mixed liquid is amplified according to temperature control by a thermal cycler. The thermal cycler performs thermal cycle treatment of repeating one cycle changing temperature of the mixed liquid to a plurality of different temperatures, multiple times on the mixed liquid.

In step S33, an emulsion containing a magnetic particle, a reagent 11 for an amplification reaction, and DNA, is formed (refer to Fig. 31(B)). That is, a droplet 14 containing a mixed liquid of a reagent 11 containing a magnetic particle, polymerase, and the like, and DNA, is formed, and a large number of droplets 14 is dispersed into a dispersion medium 15. A primer 12 for nucleic acid amplification is applied to a surface of each of the magnetic particles confined in the droplet 14. The droplet 14 is formed such that each of the magnetic particle and a target DNA molecule is contained in the droplet 14 to the extent of about one piece. The dispersion medium 15 is immiscible to the mixed liquid. In the present example, the mixed liquid is water-based, and the dispersion medium is oil-based. The dispersion medium 15 is oil, for example.

In step S34, DNA binds to the primer 12 on the magnetic particle to be amplified in each of the droplets 14 of the emulsion according to temperature control by the thermal cycler (emulsion PCR)(refer to Fig. 31(C)). This causes the target DNA molecule to be amplified in each of the droplets 14.

After DNA is amplified on a magnetic particle, an emulsion is broken down in step S35 and the magnetic particle containing the amplified DNA is extracted from the droplet 14 (emulsion break). As the reagent 16 for breaking down the droplet 14, one or more kinds of reagent containing alcohol, surfactant, and the like are used.

In step S36, the magnetic particle extracted from the droplet 14 is cleaned in a BF separation step (primary cleaning). The BF separation step is a treatment step in which the magnetic particle containing the amplified DNA is passed through a cleaning liquid while being collected by a magnetic force so that unnecessary substances adhering to the magnetic particle is removed. In the primary cleaning step, a cleaning liquid containing alcohol is used, for example. The alcohol not only removes an oil film on the magnetic particle, but also denaturalizes amplified double stranded DNAto a single strand.

After the cleaning, the DNA denaturalized to a single strand on the magnetic particle is caused to bind to the marking substance 17 for detection (hybridization) in step S37 (refer to Fig. 31(D)). The marking substance 17 is a fluorescent substance, for example. The marking substance 17 is designed to specifically bind to the DNA to be detected.

In step S38, the magnetic particle binding to the marking substance 17 is cleaned in the BF separation step (secondary cleaning). The secondary BF separation step is performed by treatment similar to that of the primary BF separation step. In the secondary cleaning step, phosphate buffered saline (PBS) is used as a cleaning liquid, for example. The PBS removes an unreacted marking substance (including a marking substance that is nonspecifically adsorbed to the magnetic particle) that has not bound to DNA.

In step S39, the DNA is detected with a hybridized marking substance 17. The DNA is detected with a flow cytometer, for example. In the flow cytometer, the magnetic particle containing the DNA binding to the marking substance 17 flows through a flow cell, and the magnetic particle is irradiated with a laser beam. Then, fluorescence emitted from the marking substance 17 by being irradiated with the laser beam is detected.

The DNA may be detected by image processing. For example, the magnetic particle containing the DNA binding to the marking substance 17 is dispersed on a flat slide or a flow channel, and the dispersed magnetic particle is imaged by a camera unit. The number of the magnetic particles emitting fluorescence is counted on the basis of the imaged image.

### (Configuration example of flow channel of specimen treatment chip)

Fig. 32 illustrates a configuration example of a flow channel of the specimen treatment chip 100 used in an emulsion PCR assay.

The specimen treatment chip 100 of Fig. 32 is composed of a fluid module 200 having a plurality of kinds of function. The plurality of kinds of function of the fluid module corresponds to a configuration of flow channels formed in the fluid module 200. In the example of Fig. 32, the fluid module 200 includes a first flow channel 110, a second flow channel 120, and a third flow channel 130. In the example of Fig. 32, the fluid module 200 further includes a fourth flow channel 140, a fifth flow channel 150, and a sixth flow channel 160. In the example of FIG. 32, each of the flow channels is connected in series in the order of the sixth flow channel 160, the first flow channel 110, the second flow channel 120, the third flow channel 130, the fourth flow channel 140, and the fifth flow channel 150 from an inflow side of a liquid containing a nucleic acid 10.

The first flow channel 110, the second flow channel 120, and the third flow channel 130 are connected so that a liquid containing the nucleic acid 10 flows continuously, for example. As a result, as compared with a case of performing intermittent liquid feeding in which a liquid is stopped to flow in the course of flowing through from the first flow channel 110 to the third flow channel 130, allowing a liquid to continuously flow from the first flow channel 110 to the third flow channel 130 enables time required for specimen treatment to be easily shortened. In the example of Fig. 32, the liquid may be caused to continuously flow through all the flow channels from the sixth flow channel 160 to the fifth flow channel 150. In addition, in the example of Fig. 32, the liquid may be caused to continuously flow in the first flow channel 110 to the third flow channel 130, and a flow of the liquid may be temporarily stopped for specimen treatment in any one or more of the fourth flow channel 140, the fifth flow channel 150, and the sixth flow channel 160, for example.

Liquid such as DNA being an object component, a reagent, and the like sequentially flow through a flow channel in each fluid module on the specimen treatment chip 100 to perform an emulsion PCR assay. In the example of Fig. 32, the sixth flow channel 160 is used to perform Pre-PCR, and the first flow channel 110 is used to form (emulsify) a droplet 14. The second flow channel 120 is used to amplify a nucleic acid (PCR), and the third flow channel 130 is used to break down (emulsion break) the droplet 14. The fourth flow channel 140 is used to perform treatment (cleaning) for collecting carriers 13, and the fifth flow channel 150 is used to perform binding (hybridization) of an amplification product and the marking substance 17.

As described above, in the configuration example of Fig. 32, the specimen treatment chip 100 further includes the fourth flow channel 140 for collecting carriers 13 extracted from respective droplets 14 by breaking down the droplets 14, and the fifth flow channel 150 for causing an amplification product on each of the collected carriers 13 and the marking substance 17 to bind to each other. As a result, it is possible to not only form a droplet 14, amplify a nucleic acid, and break down the droplet 14, but also perform treatment for collecting carriers 13 and treatment for causing an amplification product and the marking substance 17 to react with each other, in the corresponding flow channels of the specimen treatment chip 100. For example, as compared with a configuration in which droplets 14 are dispensed into a large number of wells formed in the specimen treatment chip 100, each treatment can be performed simply by causing a liquid to flow through the corresponding flow channels, whereby a series of treatment of an emulsion PCR assay can be easily and quickly performed.

Hereinafter, a configuration of each flow channel will be described according to the order in which a liquid containing a nucleic acid flows.

### <Sixth flow channel>

Fig. 33 illustrates a configuration example of the sixth flow channel 160 used for Pre-PCR. The sixth flow channel 160 includes a channel 161, connection portions 162a and 162b for injecting a reagent and a specimen, and a connection portion 162c for discharging a liquid. The channel 161 is formed in a diamond shape, for example, to control a flow rate of liquid.

For example, DNA extracted in pretreatment is injected from the connection portion 162a, and a PCR amplification reagent is injected from the connection portion 162b. The mixed liquid of the DNA and the reagent is controlled by the heater 541 for temperature in the course of flowing through the channel 161. The DNA and the reagent react with each other with temperature control to amplify the DNA. The liquid containing the amplified DNA is fed to the adjacent fluid module 200 through the connection portion 162c.

For example, when Pre-PCR is performed as pretreatment by an external device, the sixth flow channel 160 may not be provided in the specimen treatment chip 100.

### <First flow channel>

Fig. 34 illustrates a configuration example of the first flow channel 110 used for forming an emulsion. The first flow channel 110 includes a channel 111, connection portions 112a, 112b, 112c, and 112d into which a liquid such as a specimen or a reagent is injected, and a connection portion 112e from which the liquid is discharged. The first flow channel 110 may include only one of the connection portions 112a and 112b. The channel 111 includes an intersection 113 at which at least two channels intersect.

For example, a liquid containing DNA amplified by Pre-PCR flows from the connection portion 112a, and a liquid containing a carrier 13 and a reagent 11 for an amplification reaction is injected from the connection portion 112b. In the present example, the carrier 13 is a magnetic particle. The liquids injected from the respective connection portions 112a and 112b are mixed in the channel 111 to flow into the intersection 113. The magnetic particle has a particle diameter of 0.5 µm to 3 µm, for example. The pump 521 applies pressure P (P is 1000 mbar or more and 10000 mbar or less) to feed liquid to the connection portions 112a and 112b.

For example, a dispersion medium 15 is injected from connection portions 112c and 112d. The dispersion medium 15 is oil for forming an emulsion, for example. The injected oil flows into the intersection 113 through different paths. The pump 521 applies pressure P (P is 1000 mbar or more and 10000 mbar or less) to feed oil to the connection portion 112a. At the intersection 113, a flow of the mixed liquid and a flow of the dispersion medium 15 intersect with each other to form an emulsion.

To increase resistance to the pressure applied by the pump 521, it is preferable that the substrate 300 has a thickness d of 2 mm or more in the present embodiment. For example, liquid under a pressure of about 8000 mbar may cause a crack in the substrate 300 when it is too thin. The substrate 300 with a thickness d of 2 mm or more prevents a crack in the substrate 300.

Fig. 35 illustrates a configuration example of the intersection 113.

In the example of Fig. 35, the first flow channel 110 includes a first channel 111a through which a mixed liquid flows, second channels 111b through which a dispersion medium 15 immiscible to the mixed liquid flows, and an intersection 113 at which the first channel 111a and the second channels 111b intersect. This makes it possible to efficiently form droplets 14 of the mixed liquid by applying a shearing force caused by a flow of the dispersion medium 15 to a flow of the mixed liquid.

In the example of Fig. 35, the intersection 113 is formed so that the first channel 111a and the second channels 111b are orthogonal to each other. At the intersection 113, the first channel 111a and a third channel 111c connected to the connection portion 112e for discharge are linearly formed, and the second channels 111b are connected so as to be orthogonal to the first channel 111a and the third channel 111c. In addition, at the intersection 113, the two second channels 111b intersect each other across the first channel 111a from both sides.

Each of the channels 111a to 111c has a width W1 of 5 µm or more and 100 µm or less at the intersection 113, for example. As a result, the channels 111a to 111c can be prevented from being clogged while generation speed of droplets 14 (or the number of droplets generated per unit time) is sufficiently secured. In the present embodiment, the channels 111a to 111c each have a width W1 of about 20 µm.

A mixed liquid of DNA and a reagent flows through the first channel 111a to flow into the intersection 113. Oil flows into the intersection 113 from the upper and lower second channel 111b in Fig. 35. The mixed liquid is divided into droplets 14 by a shear force generated by being pressed by the oil at the intersection 113. The divided droplets 14 are surrounded by the oil flowing into the intersection 113 to form an emulsion. The emulsion formed from a flow of a specimen flows forward through the third channel 111c to be fed into the adjacent second flow channel 120 through the connection portion 112e.

The control unit 530 controls supply pressure to be applied to the mixed liquid and the dispersion medium 15 by the liquid feeder 520 so that the mixed liquid and the dispersion medium 15 are fed into the first flow channel 110 by the applied pressure to form the droplets 14. This makes it possible to continuously form the droplets 14 in the dispersion medium 15 to which pressure is applied, as compared with a configuration in which the mixed liquid is dropped into the dispersion medium 15 stored in a well, for example. As a result, the droplets 14 can be generated at high speed.

For example, the mixed liquid of DNA and the reagent flows into the intersection 113 at a flow rate of 0.4 µL/min to 7 µL/min, and the oil flows into the intersection 113 at a flow rate of 1 µL/min to 50 µL/min. The flow rate is controlled by pressure applied by the pump 521. For example, when the mixed liquid of DNA and the reagent, and the oil are caused to flow into the intersection 113 at flow rates of 2 µL/min (about 5200 mbar) and 14 µL/min (about 8200 mbar), respectively, droplets 14 of about 10 million pieces/min are formed.

The control unit 530 controls pressure of the liquid feeder 520 so that the droplets 14 are formed at a rate of 0.6 million pieces/minute or more and 18 million pieces/minute or less, for example. Forming the droplets 14 at such a high speed enables time required for specimen treatment to be shortened. When the droplets 14 are formed at high speed, it is difficult to precisely control a particle diameter of each droplet 14 and a variation of generation speed of the droplets 14. In contrast, in the present embodiment, the need for precise control of a particle diameter, generation speed, and the like of the droplet 14 can be eliminated by breaking down the droplet 14 after being amplified in the third flow channel 130, so that droplet formation can be speeded up without affecting accuracy of nucleic acid detection.

To form droplets 14 at high speed as described above, it is necessary to apply a high pressure to the specimen treatment chip 100. As described above, the substrate 300 capable of withstanding high pressure can be easily obtained by setting the thickness d of the substrate 300 and selecting the material of the substrate 300. In addition, use of the through hole 310 provided in the substrate 300 as the liquid injection port 101 enables pressure resistance performance of the liquid injection port 101 of the specimen treatment chip 100 to be easily improved. Forming the through hole 310 in a simple shape such as a through hole in the thickness direction is also effective in improving the pressure resistance performance.

In the example of Fig. 35, the intersection 113 is formed in a cross shape by a total of four channels 111 including one first channel 111a into which mixed liquid flows, two second channels 111b into which oil flows, and one third channel 111c through which an emulsion flows out. In the example of Fig. 36, the intersection 113 is formed in a T shape by three channels 111. In the example of Fig. 36, the mixed liquid flows from one first channel 111a, and oil flows from one second channel 111b. Due to a shear force generated by a flow of the oil, the mixed liquid turns into droplets in the oil to form an emulsion. The emulsified flow of a specimen flows out from one third channel 111c.

### <Second flow channel>

Fig. 37 illustrates a configuration example of the second flow channel 120 used for emulsion PCR. The second flow channel 120 includes a channel 121, a connection portion 122a into which a liquid flows, and a connection portion 122b through which a liquid is discharged.

The second flow channel 120 is formed such that a droplet 14 alternately passes through a plurality of temperature zones TZ, for example. This enables thermal cycle processing to be performed merely by feeding the droplet 14 in the second flow channel 120. That is, as compared with a configuration in which the droplet 14 is stopped to flow in the second flow channel 120 and temperature of the heater 541 is periodically changed, the processing can be performed quickly, for example. In addition, operation control in the specimen treatment apparatus 500 handling the specimen treatment chip 100 can also be simplified. The number of temperature zones TZ may be any number other than three.

In the example of Fig. 37, the channel 121 has a meandering structure such that it passes through a plurality of temperature zones TZ1 to TZ3 formed by the heater 541 multiple times. The number of times that the channel 121 passes through the respective temperature zones TZ1 to TZ3 corresponds to the number of thermal cycles. That is, the second flow channel 120 is formed in such a shape as to extend back and forth in the plurality of temperature zones TZ by the number corresponding to the number of thermal cycles across the plurality of temperature zones TZ. As a result, the thermal cycle processing can be easily performed by the desired number of cycles by only causing an emulsion containing the droplet 14 to pass through the second flow channel 120.

The number of thermal cycles of the emulsion PCR is set to about 40, for example. Thus, while illustrated in a simplified manner in Fig. 37, the channel 121 is formed in a reciprocating shape or meandering shape for the number of times corresponding to the number of cycles so as to traverse the temperature zones TZ1 to TZ3 about 40 times.

As illustrated in Fig. 37, DNAin each droplet 14 is amplified in the course of flowing through the channel 121. The droplet 14 containing the amplified DNA is fed to the adjacent third flow channel 130 through the connection portion 122b.

### <Third flow channel>

Fig. 38 illustrates a configuration example of the third flow channel 130 used for breaking down an emulsion. The third flow channel 130 has a function of mixing a plurality of kinds of liquid. The third flow channel 130 includes a channel 131, connection portions 132a, 132b, and 132c into which a reagent 16 for breaking down an emulsion and a droplet flows, and a connection portion 132d through which a liquid is discharged.

For example, an emulsion having undergone the emulsion PCR step flows from the connection portion 132b, and the reagent 16 for breaking down a droplet flows from the connection portions 132a and 132c. The emulsion and the reagent 16 for breaking down a droplet are mixed in the course of flowing through the channel 131 to break down droplets 14 in the emulsion. The channel 131 is formed in such a shape that promotes mixing of liquid.

For example, the third flow channel 130 has a curved shape to generate a turbulent flow for mixing the droplet 14 and the reagent 16 for breaking down the droplet. This enables the liquid droplet 14 and the reagent 16 for breaking down a droplet to be agitated when passing through the curved third flow channel 130, so that mixing can be promoted.

Specifically, the third flow channel 130 has a meandering shape, for example. This enables a large number of curved or bent portions to be provided in the third flow channel 130, so mixing can be promoted more effectively. In the configuration example of Fig. 38, the third flow channel 130 includes a plurality of bent portions 133 and a plurality of linear portions 134 connecting between the corresponding bent portions 133. In other words, the third flow channel 130 has a folded structure in which the linear portion 134 is folded back to the opposite side at the bent portion 133. This causes a liquid to alternately pass through the linear portion 134 and the bent portion 133, so that repeated turbulent flow can be generated to agitate the liquid. As a result, mixing can be promoted even more effectively. A magnetic particle extracted from the droplet 14 is fed to the adjacent fourth flow channel 140 through the connection portion 132d.

### <Fourth flow channel>

Fig. 39 illustrates a configuration example of the fourth flow channel 140 used in the cleaning step (primary cleaning). The fourth flow channel 140 includes a channel 141, connection portions 142a and 142b into which a liquid flows, and connection portions 142c and 142d from which a liquid is discharged.

The fourth flow channel 140 includes a linear portion 143 for capturing a magnetic particle with a magnetic force to move the magnetic particle back and forth in a direction along the fourth flow channel 140, for example. This enables the magnetic particle to be easily collected and cleaned in the linear portion 143. In addition, when magnetic particles are moved back and forth in a cleaning liquid in the linear portion 143, the magnetic particles can be prevented from sticking to each other in a massive form. The linear portion 143 has a shape extending linearly in a predetermined direction, such as a substantially rectangular shape, for example. In the example of Fig. 39, the entire channel 141 is a linear portion 143. The linear portion 143 may be formed as a part of the channel 141.

In the example of Fig. 39, the connection portions 142a and 142b on an inflow side are connected to one end side of the linear portion 143, and the connection portions 142c and 142d on a discharge side are connected to the other end side of the linear portion 143. One of the connection portions 142a and 142b is used to supply a cleaning liquid, and the other of the connection portions 142a and 142b is used to supply magnetic particles. One of the connection portions 142c and 142d is used to discharge the cleaning liquid, and the other of the connection portions 142c and 142d is used to feed the magnetic particles to the next flow channel. This enables operations of feeding magnetic particles into the fourth flow channel 140, discharging a cleaning liquid while allowing it to flow into the fourth flow channel 140, and feeding cleaned magnetic particles from the fourth flow channel 140, by allowing a liquid to flow in the same direction. As a result, no back flow of a liquid occurs to enable the cleaning step to be performed efficiently.

In addition, in the example of Fig. 39, the linear portion 143 has a channel width W3 larger than a channel width W2 of the connection portion 142a for allowing a liquid to flow in therethrough. This enables the linear portion 143 to have a wide shape capable of bringing magnetic particles into sufficient contact with a cleaning liquid. As a result, cleaning efficiency can be improved.

Fig. 40 illustrates an example of operation of cleaning and concentrating magnetic particles in the fourth flow channel 140. From the connection portion 142a, a liquid containing magnetic particles flows into the channel 141. The magnetic particles in the liquid are concentrated by a magnetic force of the magnet 640. The magnet 640 can reciprocate in a longitudinal direction of the linear portion 143. The magnetic particles are agglomerated while moving back and forth in the linear portion 143 in accordance with reciprocating motion of the magnet 640.

From the connection portion 142b, a cleaning liquid is supplied. The cleaning liquid continuously flows from the connection portion 142b toward the connection portion 142d while passing through the linear portion 143. The connection portion 142d serves as a drain for discharging the cleaning liquid. The magnetic particles move back and forth in a flow of the cleaning liquid in the linear portion 143 in accordance with motion of the magnet 640 to perform cleaning treatment. The magnetic particles move back and forth in the linear portion 143 in accordance with motion of the magnet 640 to be prevented from sticking to each other in a massive form.

In the primary cleaning step, a cleaning liquid containing alcohol is used. The primary cleaning using the cleaning liquid removes an oil film on the magnetic particle to denaturalize amplified double stranded DNA to a single strand. The magnetic particles having being cleaned and concentrated are discharged from the connection portion 142c to be fed to the adjacent fifth flow channel 150.

### <Fifth flow channel>

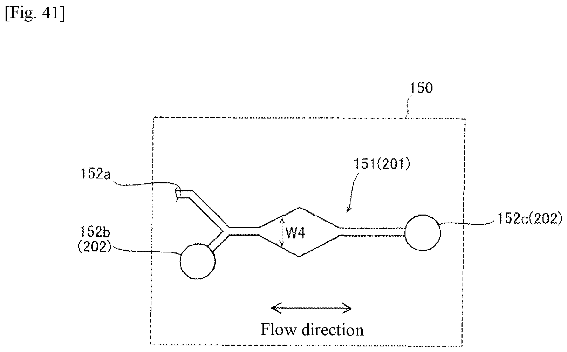

Fig. 41 illustrates a configuration example of the fifth flow channel 150 used in a hybridization step. The magnetic particles are mixed with a reagent containing a marking substance in the fifth flow channel 150 to be subjected to a thermal cycle. The fifth flow channel 150 may have the same configuration as the sixth flow channel 160 of Fig. 33. That is, the fifth flow channel 150 includes connection portions 152a and 152b on one side for allowing a liquid to flow in therethrough, a connection portion 152c on the other side for allowing a liquid to flow out therethrough, and a channel 151 that connects between the connection portions 152a and 152b on an inflow side, and the connection portion 152c on an outflow side.

In the configuration example of Fig. 41, a liquid containing magnetic particles is fed from the connection portion 152a, and a reagent containing a marking substance 17 is injected from the connection portion 152b, for example. The thermal cycle causes DNA on the magnetic particle and the marking substance 17 to bind to each other.

In the configuration example of Fig. 41, the channel 151 of the fifth flow channel 150 has a channel width W4 varying along a flow direction. That is, the channel 151 gradually increases in width from its upstream side along the flow direction, and gradually decreases in width from its substantially central position toward its downstream side. In the example of Fig. 41, the channel 151 has a rhombic shape. Changing the channel width W4 along the flow direction as described above enables a flow rate of a liquid inside the channel 151 to be controlled. As a result, the flow rate thereof can be controlled so as to be suitable for causing an amplification product of the nucleic acid 10 and the marking substance 17 to bind to each other, the flow rate being different from flow rates in other flow channels.

The secondary cleaning step after hybridization (binding) with the marking substance 17 may be performed in the fifth flow channel 150. For example, a cleaning liquid is injected from the connection portion 152b while a magnetic particle is magnetically collected in the channel 151 by the magnet 640 (refer to Fig. 40), in Fig. 41. In the secondary cleaning step, PBS is used as the cleaning liquid. The secondary cleaning using the cleaning liquid removes an unreacted marking substance 17 (including a marking substance nonspecifically adsorbed to a magnetic particle) that has not bound to DNA. In this case, it is preferable that the fifth flow channel 150 is also provided with a connection portion 152 on the discharge side for draining, as with the fourth flow channel 140 (refer to Fig. 39). The magnetic particle containing the marking substance 17 after the secondary cleaning is discharged from the connection portion 152c.

The fourth flow channel 140 that performs the secondary cleaning may be added downstream of the fifth flow channel 150 that performs hybridization.

### <Modification of flow channel configuration>

As another configuration example, the primary cleaning, the hybridization, and the secondary cleaning may be performed in one fourth flow channel 140 (refer to Fig. 39). In this case, a sample after emulsion breaking is injected into the channel 141 from the connection portion 142a to be magnetized by the magnet 640. From the connection portion 142b, an alcohol-containing cleaning liquid for the primary cleaning, a marking reagent for the hybridization, and a cleaning liquid (PBS) for the secondary cleaning are sequentially injected to perform treatment of each step. In this case, there is no need to provide the fifth flow channel 150 downstream of the fourth flow channel 140.

### (Description of detection step)

A magnetic particle containing the marking substance 17 after the secondary cleaning is detected by a flow cytometer, or image analysis, for example. Because a flow cytometer is used for detection, the magnetic particle containing the marking substance 17 is recovered from the specimen treatment apparatus 500 to be fed to the detection unit 550 or the flow cytometer provided outside the apparatus, for example. In the magnetic particle containing the marking substance 17, fluorescence or the like based on mark is detected by the detection unit 544 of the specimen treatment apparatus 500. In addition, the magnetic particle containing the marking substance 17 is photographed by the camera unit 545 of the specimen treatment apparatus 500, and then an image photographed is analyzed by the specimen treatment apparatus 500 or a computer connected to the specimen treatment apparatus 500.

### (Another configuration example of specimen treatment chip)

While Fig. 32 illustrates a configuration example of the specimen treatment chip 100 including the first flow channel 110, the second flow channel 120, and the third flow channel 130, another configuration example of the specimen treatment chip will be described below.

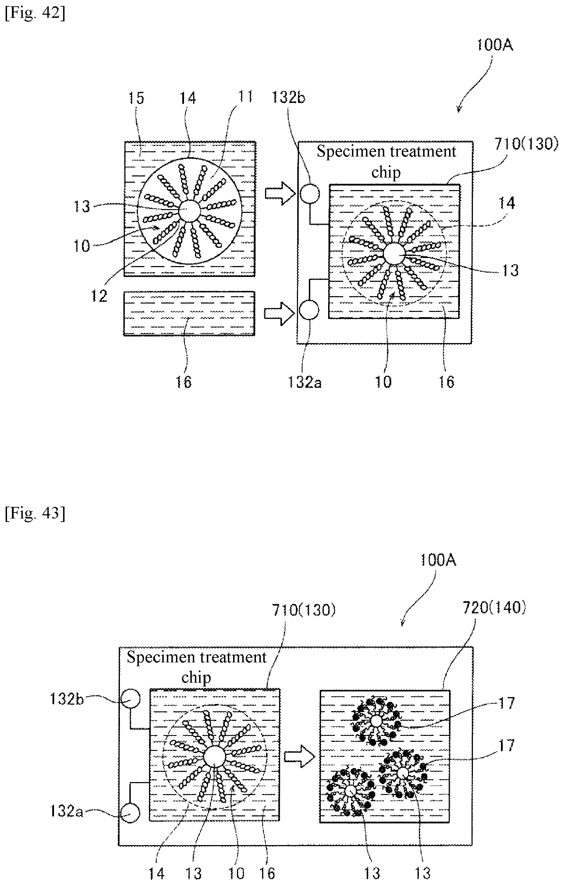

A specimen treatment chip 100A illustrated in Fig. 42 is to be installed in the specimen treatment apparatus 500 to treat a nucleic acid 10 in a specimen. The specimen treatment chip 100A includes a breaking-down flow channel 710 that is used to mix a droplet 14 containing a carrier 13 to which an amplification product of the nucleic acid 10 amplified in the droplet 14 binds, and a reagent 16 for breaking down the droplet 14, to break down the droplet 14. The breaking-down flow channel 710 may have the same function as the third flow channel 130, and may use the same configuration as that of the third flow channel 130 illustrated in Fig. 38. The specimen treatment chip 100A may be configured to include only the breaking-down flow channel 710 as illustrated in Fig. 42.

The specimen treatment chip 100 A of the present embodiment, configured as described above, allows a droplet 14 containing a carrier 13 to which an amplification product of the nucleic acid 10 amplified in the droplet 14 binds to flow into the breaking-down flow channel 710, and allows a reagent 16 for breaking down the droplet 14 to flow into the breaking-down flow channel 710. The droplet 14 and the reagent 16 for breaking down the droplet 14 are mixed in the breaking-down flow channel 710. When the droplet 14 and the reagent 16 are mixed, the droplet 14 is broken down. When the droplet 14 is broken down, the carrier 13 to which the amplification product of the nucleic acid 10 amplified in the droplet 14 binds is extracted into the breaking-down flow channel 710. When the droplet 14 after amplification treatment is broken down in the breaking-down flow channel 710 as described above, the nucleic acid 10 held by the carrier 13 can be extracted from the droplet 14.

The breaking-down flow channel 710 enables the nucleic acid 10 held by the carrier 13 to be extracted from the droplet 14, so that the nucleic acid 10 extracted from the droplet 14 can be detected by extracting and collecting the extracted carrier 13 from the specimen treatment chip 100A to cause the amplified product on the carrier 13 collected and the marking substance 17 to bind to each other, for example. As a result, unlike the case where a nucleic acid 10 is detected while being contained in a droplet 14, it is possible to treat a specimen in the specimen treatment chip 100A without requiring accurate control of a particle diameter of a droplet 14, generation speed thereof, and the like.

The breaking-down flow channel 710 includes a connection portion 132b for allowing the droplet 14 to flow in therethrough and a connection portion 132a for allowing the reagent 16 for breaking down the droplet 14 to flow in therethrough, for example.

The specimen treatment chip 100 A of the present embodiment, configured as described above, allows the droplet 14 containing the carrier 13 to which the amplification product of the nucleic acid 10 amplified in the droplet 14 binds to flow in from the connection portion 132b, and allows the reagent 16 for breaking down the droplet 14 to flow in from the connection portion 132a. Into each of the connection portion 132a and the connection portion 132b, a liquid is supplied through the liquid feeding pipe 526 of an external specimen treatment apparatus 500, or the like, for example. This makes it possible to easily feed the droplet 14 and the reagent 16 to the breaking-down flow channel 710 when a liquid is automatically fed using the external specimen treatment apparatus 500.

In the configuration example of Fig. 43, the specimen treatment chip 100A further includes a marking flow channel 720 for collecting carriers 13 extracted from droplets 14 by breaking down to cause an amplification product on each of the collected carriers 13 and a marking substance 17 for detecting an amplification product to bind to each other. The marking flow channel 720 can be configured similarly to the fourth flow channel 140. That is, the specimen treatment chip 100A may include the third flow channel 130 and the fourth flow channel 140.

In this case, a liquid containing a carrier 13 extracted from a droplet 14 by breaking down the droplet 14 in the breaking-down flow channel 710 flows into the marking flow channel 720. In the marking flow channel 720, the carrier 13 flowing in is collected, and an amplified product on the collected carrier 13 binds to a marking substance 17. As a result, a nucleic acid 10 extracted from the droplet 14 can be detected by detecting presence of the marking substance 17.

When the specimen treatment chip 100A includes the marking flow channel 720 in addition to the breaking-down flow channel 710, it is possible to perform not only treatment of extracting a carrier 13 to which an amplification product of a nucleic acid 10 amplified in a droplet 14 from the droplet 14, but also treatment of collecting the extracted carrier 13 and causing the amplification product on the carrier 13 and a marking substance 17 to bind to each other in a flow channel of the specimen treatment chip 100A. For example, as compared with a configuration in which after a droplet 14 is dispensed into a large number of wells formed in a specimen treatment chip and the droplet 14 is broken down, a carrier 13 extracted from the droplet 14 is collected in another well to perform treatment of causing an amplification product and a marking substance 17 to bind to each other, each treatment can be performed simply by causing a liquid to flow through the corresponding flow channels, whereby treatment related to an emulsion PCR assay can be easily and quickly performed in the specimen treatment chip 100A.

### <Configuration example of flow channel of specimen treatment chip>

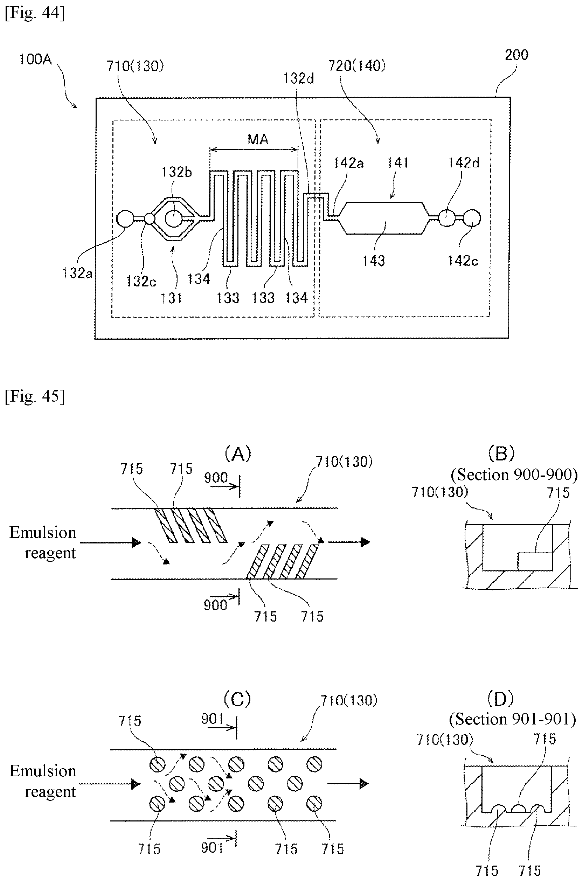

Fig. 44 illustrates a configuration example of a flow channel of the specimen treatment chip 100A. The specimen treatment chip 100A of Fig. 44 includes a fluid module 200 provided with a breaking-down flow channel 710 and a marking flow channel 720, and a substrate 300 (refer to Fig. 3) on which the fluid module 200 is disposed. In the example of Fig. 44, the fluid module 200 is formed integrally with the breaking-down flow channel 710 and the marking flow channel 720. The fluid module 200 provided with the breaking-down flow channel 710 and the fluid module 200 provided with the marking flow channel 720 may be disposed on the substrate 300. The substrate 300 includes at least one of a port 101 (refer to Fig. 7) for injecting a liquid to the fluid module 200 and a port 102 (refer to Fig. 7) for feeding a liquid from the fluid module 200. This makes it easy to inject a liquid or recover a liquid.

The breaking-down flow channel 710 and the marking flow channel 720 are connected in series in this order from an inflow side of a liquid containing a nucleic acid 10. The breaking-down flow channel 710 and the marking flow channel 720 are connected such that the liquid containing the nucleic acid 10 flows continuously. This causes a liquid after emulsion breaking treatment in the breaking-down flow channel 710 to directly flow into the marking flow channel 720 without passing through other flow channels. The liquid is caused to continuously flow, so that time required for specimen treatment can be easily shortened.

Even in the configuration example of Fig. 44, the breaking-down flow channel 710 has basically the same structure as the third flow channel 130 of Fig. 38. That is, the third flow channel 130 includes the channel 131, the connection portions 132a, 132b, and 132c into which a reagent 16 for breaking down an emulsion and a droplet flow, and the connection portion 132d through which a liquid is discharged.

In the configuration example of Fig. 44, the breaking-down flow channel 710 is provided on its one end side with a connection portion for supplying a cleaning liquid and a connection portion for supplying a marking substance 17. Here, in the configuration example of Fig. 44, the connection portion 132a of the breaking-down flow channel 710 serves as a common connection portion of the reagent 16 and the cleaning liquid for breaking down a droplet. Into the connection portion 132a, there are supplied the reagent 16 for breaking down a droplet in the breaking-down flow channel 710, the cleaning liquid containing alcohol used in the primary cleaning step (refer to Fig. 30) in the marking flow channel 720, and PBS serving as a cleaning liquid used for the secondary cleaning step (refer to Fig. 30) in the marking flow channel 720. Into the connection portion 132b, an emulsion containing droplets 14 is supplied. Into the connection portion 132c, the marking substance 17 is supplied. The cleaning liquid and the marking substance 17 pass through the breaking-down flow channel 710, and flow into the marking flow channel 720 through the connection portion 132d on the other end side of the breaking-down flow channel 710.

For example, the breaking-down flow channel 710 has a curved shape for mixing a droplet 14 and the reagent 16 for breaking down the droplet 14. The droplet 14 and the reagent 16 are agitated when passing through the curved breaking-down flow channel 710, so that mixing is promoted. The breaking-down flow channel 710 has a meander shape, for example. That is, portions of the breaking-down flow channel 710 each of which is bent in an opposite direction are alternately provided. A flow of the droplet 14 and the reagent 16 passing through the breaking-down flow channel 710 is alternately bent in an opposite direction, so that the droplet 14 and the reagent 16 are agitated. As a result, mixing is promoted. The breaking-down flow channel 710 includes a plurality of bent portions 133, and a plurality of linear portions 134 each connecting between the corresponding bent portions 133, for example. Each of the bent portions 133 forces a linear flow in the corresponding one of the linear portions 134 to be bent, so that agitation of the droplet 14 and the reagent 16 is promoted. As described above, when one of these flow channel shapes is used or a plurality of the shapes is combined to be used, the droplet 14 and the reagent 16 for breaking down the droplet can be agitated when passing through the breaking-down flow channel 710, whereby mixing of a liquid can be promoted.

The marking flow channel 720 has a structure similar to that of the fourth flow channel 140 of Fig. 39. The marking flow channel 720 includes a channel 141, a connection portion 142a into which a liquid flows, and connection portions 142c and 142d from each of which a liquid is discharged.

The marking flow channel 720 includes a linear portion 143 for moving back and forth magnetic particles captured by a magnetic force in a direction along the marking flow channel 720. This makes it possible to collect the magnetic particles in a linear flow unlike when a flow channel is bent. As a result, it is possible to easily collect and clean the magnetic particles in the linear portion 143. In addition, when magnetic particles are moved back and forth in a cleaning liquid in the linear portion 143, the magnetic particles can be prevented from sticking to each other in a massive form. The linear portion 143 and the connection portion 142a have a relationship between their flow channel widths that is similar to that of the fourth flow channel 140 of Fig. 39. The wide linear portion 143 enables a sufficient amount of the cleaning liquid to be supplied in the flow channel, so that the magnetic particles and the cleaning liquid are brought into sufficient contact with each other. As a result, cleaning efficiency can be improved.

The marking flow channel 720 is provided on its one end side with a connection portion 142a for causing a liquid containing magnetic particles extracted from droplets 14 by breaking down, the cleaning liquid, and the marking substance 17 to flow therethrough. The marking flow channel 720 is provided on its the other end side with a connection portion 142c for feeding a carrier 13 in which the marking substance 17 has reacted with an amplified product on the carrier 13, and a connection portion 142d for discharging the cleaning liquid. This causes no reverse flow of a liquid in the following operations of: feeding the magnetic particles, extracted from the droplets 14 by breaking down the droplets 14, into the marking flow channel 720; discharging the cleaning liquid while causing it to flow into the marking flow channel 720; and discharging the carrier 13 after hybridization from the marking flow channel 720. That is, each treatment can be performed by causing a liquid to flow only in the same direction, so that each treatment step can be performed efficiently.

### <Structure for promoting mixing>

The breaking-down flow channel 710 and the third flow channel 130 (refer to Fig. 38) each may be provided on its inner wall with a structure for promoting mixing of a droplet 14 and a reagent 16.. In the configuration example of Fig. 45, the breaking-down flow channel 710 is provided on its inner wall with a plurality of protrusions 715 for mixing a droplet 14 and the reagent 16 for breaking down the droplet 14. The plurality of protrusions 715 enables a turbulent flow to be generated in a flow of the droplet 14 and the reagent 16. When the flow of the droplet 14 and the reagent 16 turns into a turbulent flow, the droplet 14 and the reagent 16 are agitated to promote their mixing, whereby the droplet 14 can be efficiently broken down. As a result, a flow channel length of the breaking-down flow channel 710 can be shortened.

Figs. 45(A) and 45(B) each illustrate a first configuration example of the plurality of protrusions 715. In the first configuration example, the breaking-down flow channel 710 is provided on its inner wall with a plurality of protrusions 715 each in a tabular shape. The protrusion 715 is formed so as to project inward from an inner surface of the flow channel. The protrusion 715 is inclined toward a downstream side of the flow channel. The plurality of protrusions 715 is disposed substantially parallel to each other side by side at a predetermined interval along the flow channel. The plurality of protrusions 715 is provided on one inner side surface of the flow channel, and is provided on the other inner side surface opposed to the one inner side surface of the flow channel. When a flow of the droplet 14 and the reagent 16 is disturbed so as to meander in the flow channel by the plurality of protrusions 715, the flow of the droplet 14 and the reagent 16 is agitated.

Figs. 45(C) and 45(D) each illustrates a second configuration example of the plurality of protrusions 715. In the second configuration example, the breaking-down flow channel 710 is provided on its inner wall with a plurality of hemispherical protrusions 715. The protrusion 715 is formed so as to protrude upward in a hemispherical shape from an inner bottom surface of the flow channel. The plurality of hemispherical protrusions 715 is regularly or randomly disposed in the flow channel. When a turbulent flow is formed by the plurality of protrusions 715, the droplet 14 and the reagent 16 are agitated.

### <Reservoir structure>

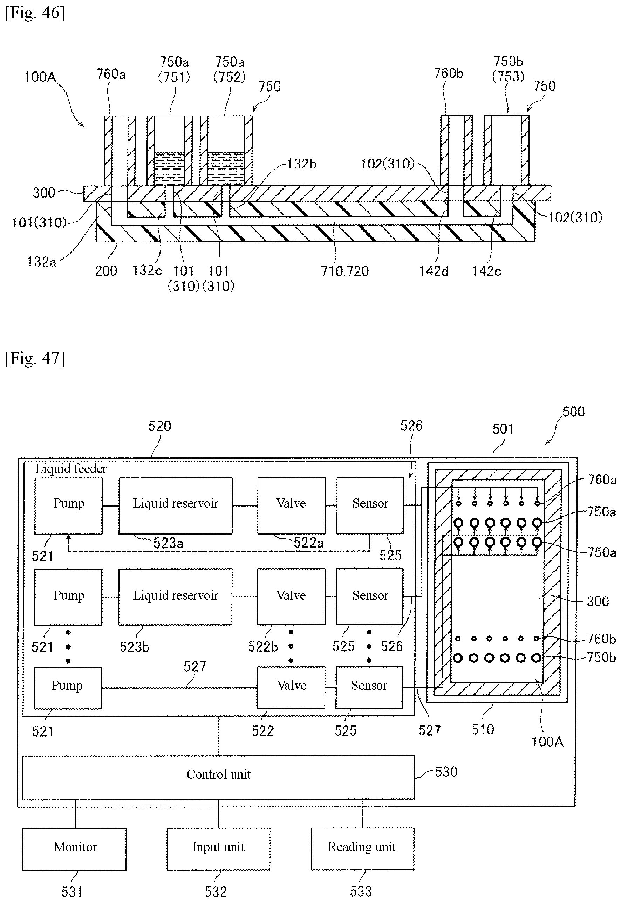

Fig. 46 illustrates a configuration example in which a reservoir 750 capable of storing liquid is provided in the specimen treatment chip 100A. In the configuration example of Fig. 46, the specimen treatment chip 100A includes a reservoir 750a for storing a liquid to be injected into the fluid module 200, or a reservoir 750b for storing a liquid to be fed from the fluid module 200.

The reservoir 750 is configured to store a droplet and a reagent to be supplied to the fluid module 200, or to store a liquid fed from the fluid module 200 after being treated in a flow channel. When the reservoir 750a for storing a liquid to be injected into the fluid module 200 is provided, a droplet to be supplied to the fluid module 200 can be easily poured into the reservoir 750a by using a pipetter. The reservoir 750a is disposed on a surface of the specimen treatment chip 100A, so that a user using a pipetter can easily pipet the liquid into the reservoir 750a. When the reservoir 750b for storing liquid to be fed out from the fluid module 200 is provided, the liquid after being treated in the flow channel can be stored until proceeding to the next treatment. Work of extracting the liquid from the reservoir 750b for the next treatment can also be easily performed using a pipetter.

In addition, a structure for storing a liquid containing a specimen can be provided in the specimen treatment chip 100A, so that a liquid containing a specimen can be fed to the fluid module 200 from the reservoir 750a without using a path for feeding liquid, like the liquid feeding pipe 526 of the specimen treatment apparatus 500, or the like. When a specimen is fed to the fluid module 200 by using the liquid feeding pipe 526 of the specimen treatment apparatus 500, the specimen remaining in the liquid feeding pipe 526 may mix with another specimen to be subjected to next treatment. Thus, when a specimen is fed to the fluid module 200 by using the liquid feeding pipe 526 of the specimen treatment apparatus 500, it is desirable to perform a process of cleaning the inside of the liquid feeding pipe 526 or to replace the liquid feeding pipe 526, every time specimen treatment is performed. In a configuration in which the specimen treatment chip 100A includes the reservoir 750a, a specimen does not need to be fed from a specimen treatment apparatus 500 side. As a result, contamination at the time of feeding a specimen to the specimen treatment chip 100A can be prevented without cleaning the liquid feeding pipe 526 or replacing the liquid feeding pipe 526. Even when liquid containing a specimen after being treated in the fluid module 200 is recovered, the liquid containing the specimen can be stored in the reservoir 750b of the specimen treatment chip 100A without being fed using the liquid feeding pipe 526 of the specimen treatment apparatus 500. Thus, it is possible to prevent contamination when a specimen is recovered from the specimen treatment chip 100A without cleaning the liquid feeding pipe 526 or replacing the liquid feeding pipe 526.

In a configuration in which the reservoir 750 is provided in the specimen treatment chip 100A, a movement distance of liquid between a structure storing liquid to be supplied to the fluid module 200 and the fluid module 200 can be reduced as much as possible. For example, in a configuration in which a specimen is fed from the specimen holding unit 524 (refer to Fig. 19) of the specimen treatment apparatus 500 through the liquid feeding pipe 526, the specimen holding unit 524 and the fluid module 200 are connected to each other through the liquid feeding pipe 526 and the connector 400. As a result, a movement distance of liquid increases as compared with the configuration in which the reservoir 750 is provided in the specimen treatment chip 100A. Thus, in a configuration in which the reservoir 750 is provided in the specimen treatment chip 100A, as a movement distance of liquid decreases, responsiveness to control of feeding liquid by the specimen treatment apparatus 500 can be improved. The configuration in which the reservoir 750 is provided on a port may be applied to the configuration examples illustrated in Figs. 7 to 9.

The reservoir 750 is bonded to the substrate 300 on a port. As a bonding method of the reservoir 750, a bonding method such as solid phase bonding, or using an adhesive, similar to the bonding method of the substrate 300 and the fluid module 200 can be used. The reservoir 750 may be formed integrally with the substrate 300.

The reservoir 750a for storing liquid to be injected into the fluid module 200 is formed on the port 101 for injecting liquid to the fluid module 200. The reservoir 750b for storing liquid to be fed out from the fluid module 200 is formed on the port 102 for feeding out liquid from the fluid module 200.

The reservoir 750 has a volume suitable for liquid to be stored. The reservoir 750 is connected at its one end in a tubular shape to a port, and has the other end that is open. The reservoir 750 can store liquid supplied from the open other end.

The reservoir 750 is provided in its upper portion with an opening larger than a diameter of each of the ports 101 and 102. This facilitates access to the inside of the reservoir 750 by the pipetter. As a result, a user can extremely easily dispense liquid to be used in the fluid module 200 into the reservoir 750a by using a pipetter. Likewise, a user can extremely easily suck liquid after being treated from the reservoir 750b by using a pipette.

In the configuration example of Fig. 46, a reservoir 751 serves as the reservoir 750a that stores a reagent containing a marking substance 17, and a reservoir 752 serves as the reservoir 750a that stores an emulsion containing an droplet 14, for example. A reservoir 753 serves as the reservoir 750b for recovering a sample that is fed out through the breaking-down flow channel 710 and the marking flow channel 720 of the fluid module 200.

The specimen treatment chip 100A is provided on its port with an injection pipe 760a for injecting liquid into the specimen treatment chip 100A, or a feeding-out pipe 760b for feeding out liquid from the specimen treatment chip 100A. The injection pipe 760a is joined to the substrate 300 on the port 101 for injecting liquid, and the feeding-out pipe 760b is joined to the substrate 300 on the port 102 for feeding out liquid. In the configuration example of Fig. 46, the reagent 16 for breaking down a droplet, the cleaning liquid used in the primary cleaning step, and the cleaning liquid used in the secondary cleaning step are supplied through the injection pipe 760a. The cleaning liquid having passed through the marking flow channel 720 is discharged through the feeding-out pipe 760b. The configuration in which the injection pipe 760a and the feeding-out pipe 760b are provided on the respective ports may be applied to the configuration examples illustrated in Figs. 7 to 9.

The substrate 300 may have the same structure as that illustrated in each of Figs. 3 and 4. That is, the substrate 300 has a thickness "d" of 1 mm or more and 5 mm or less, for example. This enables the substrate 300 to be formed to have a sufficiently large thickness as compared with a flow channel height (on the order of 10 µm to 500 µm) of the flow channel formed in the fluid module 200. As a result, sufficient pressure resistance performance can be easily secured for the substrate 300. The fluid module 200 and the substrate 300 each are made of glass or a resin composition. In the case where the substrate 300 is made of glass, sufficient pressure resistance performance can be secured for the substrate 300 even when pressure of the liquid to be supplied to the fluid module 200 is increased according to a treatment step. The resin composition is composed of a plurality of components including at least a resin. In the case where the substrate 300 is made of a resin composition, the same material as the fluid module 200 can be used, for example, thereby facilitating bonding in that case. Examples of the resin composition include polycarbonate (PC), polystyrene (PS), polydimethylsiloxane (PDMS), polymethyl methacrylate resin (PMMA), cycloolefin copolymer (COC), cycloolefin polymer (COP), and other resin compositions.

The substrate 300 includes at least one of the port 101 for injecting a liquid into the fluid module 200 and the port 102 for feeding a liquid from the fluid module 200. When the port 101 is provided, a liquid is fed to the fluid module 200 from the outside through the port 101. This eliminates the need to provide a structure for feeding liquid to a specimen treatment chip 100A side, so that the specimen treatment chip 100A can be downsized. When the port 102 is provided, a liquid treated in the fluid module 200 flows out through the port 102. This enables a liquid containing a marked nucleic acid 10 to be easily extracted when a nucleic acid is detected outside the specimen treatment chip 100A, for example.

For example, at least one of the port 101 for injecting a liquid into the fluid module 200 and the port 102 for feeding a liquid from the fluid module 200 is one of the through holes 310 formed in the substrate 300. As a result, the liquid can be injected through the substrate 300 that is more likely to secure pressure resistance performance than the fluid module 200 in which the flow channel is formed. This easily enables liquid injection under sufficient pressure. The through holes 310 are formed at predetermined pitches on the substrate 300 as illustrated in Fig. 7, for example. In this case, the fluid module 200 can be disposed at an arbitrary position on a pitch unit basis on the substrate 300, and its corresponding flow channel can be connected to an arbitrary through hole 310. Thus, even when structure such as a flow channel shape of the fluid module 200 is changed, the substrate 300 does not need to be changed in structure. As a result, it is possible to flexibly deal with a design change.

The fluid module 200 is disposed at a position suitable for the through holes 310 formed at the predetermined pitches on the substrate 300, and includes connection portions 132a, 132b, 132c, 142c, and 142d connected to the corresponding through holes 310. Placement of the fluid module 200 on the substrate 300 and the position of each of the connection portions and the through holes 310 are similar to those in the configuration example illustrated in each of Figs. 5 and 6. Even when the fluid module 200 and the substrate 300, formed separately, are joined to each other, the connecting portions and the corresponding through holes 310 portions can be collectively connected by allowing the connecting portions of the fluid module 200 and the corresponding through holes 310 of the substrate 300 to coincide with each other.

### (Specimen treatment apparatus)

Fig. 47 illustrates a configuration example of a specimen treatment apparatus 500 for treating a nucleic acid 10 in a specimen using the specimen treatment chip 100A illustrated in Fig. 46.

The specimen treatment apparatus 500 includes an installation unit 510 for installing the specimen treatment chip 100A, a liquid feeder 520 for supplying a liquid containing the nucleic acid 10 to the specimen treatment chip 100A, and a control unit 530. The control unit 530 of the specimen treatment apparatus 500 controls the liquid feeder 520 so as to feed the liquid containing the nucleic acid 10 into the specimen treatment chip 100A through a breaking-down flow channel 710 of the specimen treatment chip 100A installed in the installation unit 510.

For example, the control unit 530 controls the liquid feeder 520 so as to supply a droplet 14 containing a carrier 13 to which an amplification product of a nucleic acid 10 amplified in the droplet 14 binds, and a reagent 16 for breaking down the droplet 14 to the breaking-down flow channel 710. The droplet 14 and the reagent 16 for breaking down the droplet 14 are mixed in the breaking-down flow channel 710, and then the droplet 14 is broken down by action of the reagent 16. When the droplet 14 is broken down, the carrier 13 to which the amplification product of the nucleic acid 10 amplified in the droplet 14 binds is extracted into the breaking-down flow channel 710. When the droplet 14 after amplification treatment is broken down in the breaking-down flow channel 710 as described above, the nucleic acid 10 held by the carrier 13 can be extracted from the droplet 14.

The nucleic acid 10 held by the carrier 13 can be extracted from the droplet 14 by causing the droplet to pass through the breaking-down flow channel 710. This enables the nucleic acid 10 extracted from the droplet 14 to be detected by extracting and collecting the extracted carrier 13 from the specimen treatment chip 100A to cause the amplified product on the carrier 13 collected and the marking substance 17 to bind to each other, for example. As a result, unlike the case where a nucleic acid 10 is detected while being contained in a droplet 14, it is possible to treat a specimen in the specimen treatment chip 100A without requiring accurate control of a particle diameter of a droplet 14, generation speed thereof, and the like.

Each of liquid reservoirs 523a, 523b,... of the specimen treatment apparatus 500 stores a reagent and a cleaning liquid to be supplied to the breaking-down flow channel 710 and the marking flow channel 720 of the fluid module 200. That is, the reagent 16 for breaking down a droplet, the cleaning liquid used in the primary cleaning step, the cleaning liquid used in the secondary cleaning step, and the like are stored in separate liquid reservoirs. In the present configuration example, instead of providing the specimen holding unit 524 (refer to Fig. 10) that holds a specimen in the specimen treatment apparatus 500, a reservoir 750 serving as a specimen holding unit is provided on a port 101 of the specimen treatment chip 100A.

In addition to the specimen holding unit 524, the reservoir 750 may be disposed on the port 101 of the specimen treatment chip 100A in place of a liquid reservoir 523 for another reagent. This enables a specimen and a reagent to be directly injected into a flow channel from above the port 101.

In the configuration in which the reservoir 750 is provided in the specimen treatment chip 100A, the specimen treatment apparatus 500 includes an air passage 527 between a pump 521 and a valve 522, as well as between the valve 522 and the reservoir 750. The pump 521 can cause a liquid to flow into a flow channel by applying pressure to the reservoir 750 through the air passage 527.

The control unit 530 controls opening and closing of the respective valves 522 of the liquid feeder 520 to feed a liquid in the specimen treatment chip 100A into the breaking-down flow channel 710 by using pressure. The control unit 530 controls timing of opening of the valve 522 on the basis of an elapsed time from an injection of a liquid into the specimen treatment chip 100A, or the injection amount of the liquid into the specimen treatment chip 100A, for example. This enables a plurality of kinds of liquid and reagent to be easily supplied to the breaking-down flow channel 710 in the specimen treatment chip 100A at a desired timing by simply controlling opening and closing timing of each valve 522.

### <Connection structure to specimen treatment chip>

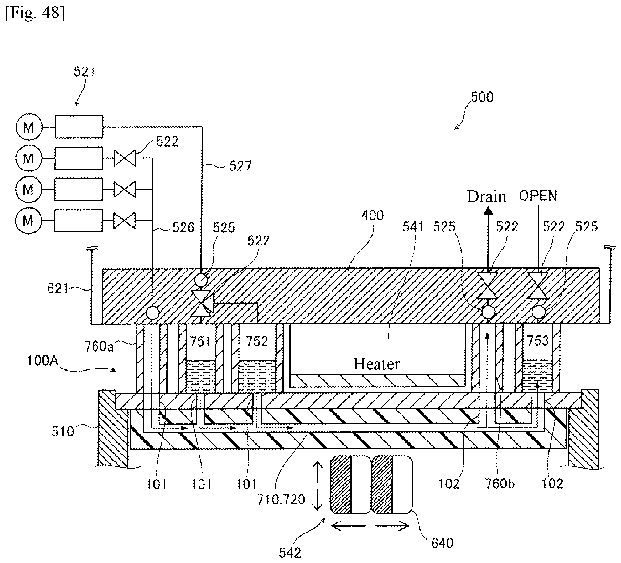

Fig. 48 illustrates a specimen treatment chip 100A installed in an installation unit 510, and a connector 400 provided in a lid 621 corresponding to the installation unit 510. The connector 400 may be configured as a manifold in which a plurality of liquid feeding pipes 526 and air passages 527 are formed. It is possible to form the connector 400 capable of individually connecting all reservoirs 750, an injection pipe 760a, and a feeding-out pipe 760b, of the specimen treatment chip 100A, to the corresponding air passages 527 and liquid feeding pipes 526.
When the lid 621 is closed, the liquid feeding pipes 526 and the air passages 527 are collectively connected to the corresponding portions of the specimen treatment chip 100A with the connector 400.

The connector 400 may include a valve 522 or a flow rate sensor 525. The connector 400 of Fig. 48 is provided in its inside with valves 522 and flow rate sensors 525. This enables a path between each of the valves 522 and the flow channel of the specimen treatment chip 100A to be shortened, so that it is possible to improve responsiveness of control of feeding liquid by opening and closing of each of the valves 522.

The connector 400 includes one of the valves 522, connected to a reservoir 751 that stores a reagent containing a marking substance 17, one of the valves 522, connected to a reservoir 752 that stores an emulsion containing a droplet 14, and the flow rate sensors 525 each of which is disposed in a path to the corresponding one of the valves 522. In Fig. 48, each of the valves 522 is a three-way valve, so that pressure from the pump 521 can be selectively supplied to the reservoir 751 or the reservoir 752.

The connector 400 includes one of the flow rate sensors 525, for the liquid feeding pipes 526 connected to the injection pipe 760a. The connector 400 is connected to a liquid feeding pipe 526 for supplying a reagent 16 for breaking down a droplet, a liquid feeding pipe 526 for supplying a cleaning liquid to be used in the primary cleaning step, and a liquid feeding pipe 526 for supplying a cleaning liquid to be used in the secondary cleaning step. The connector 400 is connected to the injection pipe 760a while integrating the liquid feeding pipes 526 into one system. The specimen treatment apparatus 500 can selectively supply each liquid to the injection pipe 760a by opening and closing the corresponding one of the valves 522 of the respective liquid feeding pipes 526.

In addition, the connector 400 includes one of the valves 522 as well as one of the flow rate sensors 525, connected to a reservoir 753 for recovering a sample collection, and one of the valves 522 as well as one of the flow rate sensors 525, connected to the feeding-out pipe 760b. The specimen treatment apparatus 500 can selectively discharge liquid in the flow channel into the reservoir 753 or the feeding-out pipe 760b by selecting the valve 522 to be opened and closed.

In the configuration in which the specimen treatment chip 100A includes the breaking-down flow channel 710 and the marking flow channel 720, the control unit 530 controls the liquid feeder 520 so as to cause a liquid containing a nucleic acid 10 to continuously flow into the breaking-down flow channel 710 and the marking flow channel 720, for example. In this case, the control unit 530 controls the liquid feeder 520 so as to cause the liquid having passed through the breaking-down flow channel 710 to directly flow into the marking flow channel 720 without stopping. As a result, as compared with a case of performing intermittent liquid feeding in which a liquid is stopped to flow in the course of flowing through the breaking-down flow channel 710 and the marking flow channel 720, time required for specimen treatment can be easily shortened.

The control unit 530 controls the liquid feeder 520 so as to cause a liquid containing a nucleic acid 10 to move back and forth in the breaking-down flow channel 710 along its flow channel. The control unit 530 causes the liquid to flow back and forth between one end side and the other end side of a range MA formed in a meander shape in the breaking-down flow channel 710 illustrated in Fig. 44, for example. This enables a droplet 14 and a reagent 16 to be mixed without increasing the number of meander sections of the breaking-down flow channel 710. As a result, the breaking-down flow channel 710 can be shortened in flow channel length, so that the specimen treatment chip 100A can be downsized.

### <Heater and magnet unit>

Fig. 48 illustrates a configuration example including a heater 541 that adjusts temperature of the marking flow channel 720 and a magnet unit 542 that applies a magnetic force to magnetic particles in the marking flow channel 720.

The heater 541 is disposed at a position overlapping with the marking flow channel 720 of the specimen treatment chip 100A. The heater 541 is disposed so as to be close to the marking flow channel 720, on an upper surface side or a lower surface side of the specimen treatment chip 100A. This enables temperature of the marking flow channel 720 to be efficiently adjusted as compared with the case where the heater 541 is disposed at a position away from the marking flow channel 720. The heater 541 heats DNA and a marking substance 17 on a magnetic particle to a predetermined temperature in the marking flow channel 720 to cause hybridization to proceed. The marking substance 17 is acquired by causing a fluorescent substance to bind to a probe composed of DNA complementary to DNA being an object component, for example. The DNA to be detected is denaturalized into a single strand while being heated to a predetermined temperature. The predetermined temperature when the hybridization is performed is typically about 70°C. Single-stranded DNA and the probe bind to each other by lowering temperature to about 40°C to 50°C, from about 70°C. For example, the heater 541 heats a liquid in the marking flow channel 720 to about 70°C, and then stops heating to lower the temperature. In the course of lowering the temperature of the liquid to room temperature, for example, the DNA and the marking substance 17 bind to each other.

The magnet unit 542 is disposed at a position overlapping with the marking flow channel 720 of the specimen treatment chip 100A. The magnet unit 542 is disposed so as to be close to the marking flow channel 720, on the upper surface side or the lower surface side of the specimen treatment chip 100A. This makes it possible to efficiently apply a magnetic force to magnetic particles in the marking flow channel 720. For example, the magnet unit 542 is disposed on a side opposite to the heater 541 across the marking flow channel 720 of the specimen treatment chip 100A. This enables both the heater 541 and the magnet unit 542 to be disposed at a position close to the marking flow channel 720.

For example, the heater 541 is disposed on the upper surface side of the specimen treatment chip 100A, and the magnet unit 542 is disposed on the lower surface side of the specimen treatment chip 100A. In this case, the heater 541 is installed on the lid 621 directly or via the connector 400. The heater 541 is provided on its lower surface side with a heat generating portion to adjust temperature of the marking flow channel 720 from an upper surface side of the specimen treatment chip 100A. The magnet unit 542 includes a movable magnet 640 on a lower surface side of the specimen treatment chip 100A installed in the installation unit 510, for example. The magnet unit 542 can move the magnet 640 in a direction along the surface of the substrate 300 as well as in a direction approaching the substrate 300 and a direction away from the substrate 300. The magnet unit 542 moves the magnet 640 during the cleaning step to move magnetic particles back and forth in a flow of the cleaning liquid in the linear portion 143 of the marking flow channel 720.

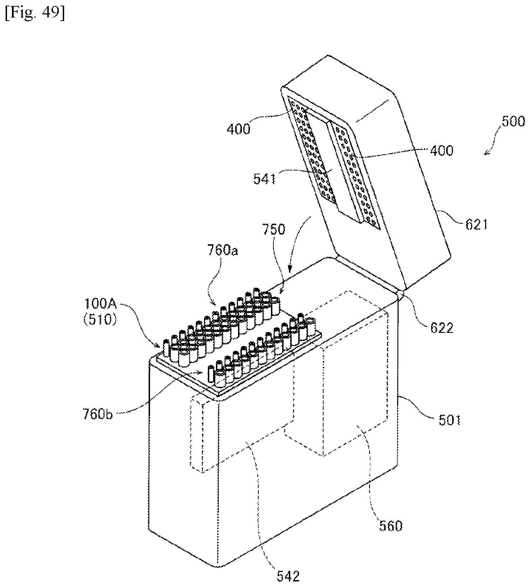

Fig. 49 is a schematic diagram illustrating appearance of the specimen treatment apparatus 500. The specimen treatment apparatus 500 includes a specimen treatment apparatus main body 501 and a lid 621 connected to the specimen treatment apparatus main body 501 with a hinge 622.

In the configuration example of Fig. 49, the installation unit 510 is disposed on an upper surface of the specimen treatment apparatus main body 501 in the shape of a box. The installation unit 510 is provided in its lower portion with a magnet unit 542. The specimen treatment apparatus main body 501 is provided in its inside with a pump unit 560 including a plurality of pumps 521. In Fig. 49, the specimen treatment chip 100A provided with a 12-channel flow channel including the breaking-down flow channel 710 and the marking flow channel 720 is set in the installation unit 510.

The lid 621 is provided on its lower surface with a connector 400 and a heater 541. When the lid 621 is closed, a reservoir 750, an injection pipe 760a, and a feeding-out pipe 760b provided in the respective channels of the 12-channel flow channel are collectively connected to the connector 400.

### (Specimen treatment method)

The emulsion PCR assay illustrated in Fig. 30 may be performed by using a plurality of kinds of specimen treatment chip.

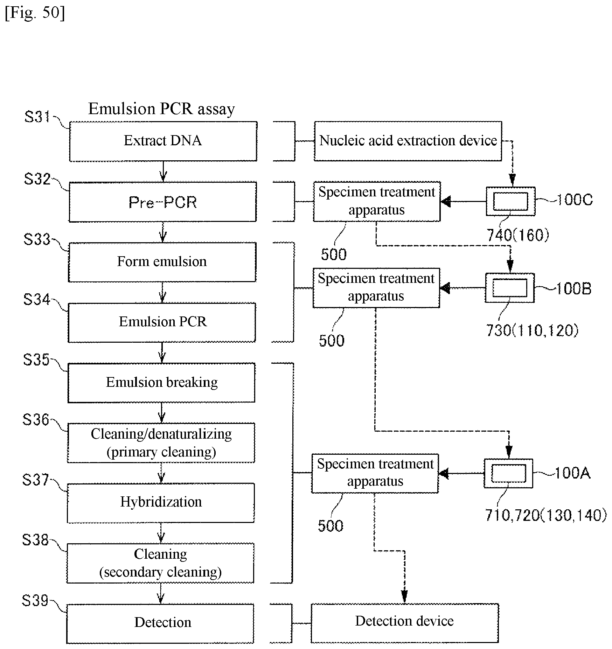

For example, a mixed liquid of a nucleic acid 10, a reagent 11 for an amplification reaction of the nucleic acid 10, and a carrier 13, and a dispersion medium 15, may be supplied to a droplet forming flow channel 730 provided in a specimen treatment chip 100B different from the specimen treatment chip 100A provided with the breaking-down flow channel 710, to form a droplet 14. In Fig. 50, an emulsion PCR assay is performed by using a first specimen treatment chip 100A including the breaking-down flow channel 710 and the marking flow channel 720, and a second specimen treatment chip 100B including the droplet forming flow channel 730.

The same structure as that of the first flow channel 110 illustrated in Fig. 34 can be applied to the droplet forming flow channel 730 of the second specimen treatment chip 100B. The second specimen treatment chip 100B may further include a second flow channel 120 for amplifying the nucleic acid in the droplet 14 to be capable of forming an emulsion and performing emulsion PCR.

According to the configuration in which an assay is performed by using the first specimen treatment chip 100A and the second specimen treatment chip 100B as described above, a flow rate of a liquid to be supplied to the flow channel of the specimen treatment chip 100A can be greatly varied. In the first flow channel 110 of the second specimen treatment chip 100B, an emulsion of droplets 14 is formed by using a mixed liquid of DNA and a reagent, and the dispersion medium 15. Thus, both flow rates of the mixed liquid and the dispersion medium 15 are added at an outlet of the first flow channel 110 to increase a flow rate in the flow channel. Meanwhile, in the breaking-down flow channel 710 of the first specimen treatment chip 100A, it is preferable to relatively reduce a flow rate of the emulsion containing droplets 14. This is because droplets 14 are efficiently broken down by reducing a flow rate of the emulsion containing the droplets 14 with respect to a reagent 16. When treatments are performed separately by using the first specimen treatment chip 100A or the second specimen treatment chip 100B, each of the treatments can be performed at a suitable flow rate as described above.

As treatment in which a flow rate largely changes, emulsion forming treatment in step S33 and emulsion breaking treatment in step S35 correspond. Thus, when an emulsion PCR assay is performed using a plurality of kinds of specimen treatment chip, it is preferable that the specimen treatment chip 100B including the droplet forming flow channel 730 (the first flow channel 110) for performing the emulsion forming treatment in step S33, and the specimen treatment chip 100A including the breaking-down flow channel 710 (the third flow channel 130) for performing the emulsion breaking treatment in step S35 are used separately.

The first specimen treatment chip 100A and the second specimen treatment chip 100B may be installed in the same specimen treatment apparatus 500, or may be installed in separate specimen treatment apparatuses 500. When the first specimen treatment chip A and the second specimen treatment chip 100B are installed in the same specimen treatment apparatus 500, the specimen treatment apparatus 500 may temporarily store droplets 14, formed by the second specimen treatment chip 100B, in the reservoir 750, and may inject the stored droplets 14 into the first specimen treatment chip 100A.

The emulsion PCR assay may be performed by further using a third specimen treatment chip 100C including a preliminary amplification flow channel 740 for amplifying DNA before a droplet is formed. The third specimen treatment chip 100C is capable of performing Pre-PCR treatment. The same structure as that of the sixth flow channel 160 illustrated in Fig. 33 can be applied to the preliminary amplification flow channel 740 of the third specimen treatment chip 100C.

In step S31, DNA is extracted by the nucleic acid extraction device, and then a specimen containing DNA is supplied to the third specimen treatment chip 100C. In step S32, the third specimen treatment chip 100C is set in the specimen treatment apparatus 500, and the Pre-PCR treatment is performed. That is, DNA extracted in pretreatment and a reagent for PCR amplification are injected into the sixth flow channel 160 serving as the preliminary amplification flow channel 740, and the DNA is amplified by a thermal cycle. After the Pre-PCR treatment, a specimen containing preliminarily amplified DNA is supplied to the second specimen treatment chip 100B.

The second specimen treatment chip 100B is set in the specimen treatment apparatus 500, and the emulsion forming treatment in step S33 and the emulsion PCR treatment in step S34 are sequentially performed. That is, a mixed liquid of DNA, a reagent 11 for the amplification reaction of DNA, and a carrier 13, and a dispersion medium 15 are supplied to the first flow channel 110 serving as the droplet forming flow channel 730 to form a droplet 14. Subsequently, the DNA in the droplet 14 is amplified by a thermal cycle in the second flow channel 120. After the emulsion PCR treatment, an emulsion containing the droplet 14 containing the amplified DNA is supplied to the first specimen treatment chip 100A.

The first specimen treatment chip 100A is set in the specimen treatment apparatus 500, and the emulsion breaking in step S35, the primary cleaning step in step S36, the hybridization in step S37, and the secondary cleaning step in step S38 are sequentially performed. That is, in the breaking-down flow channel 710, the droplet 14 and a reagent 16 for breaking down the droplet 14 are mixed, so that the droplet 14 is broken down. The carrier 13 (magnetic particle) extracted from the droplet 14 by breaking down in the breaking-down flow channel 710 is supplied to the marking flow channel 720. After the primary cleaning step is performed by collecting a magnetic particle by the magnet 640 and supplying a cleaning liquid containing alcohol, hybridization of an amplified product on the magnetic particle and the marking substance 17 is performed under temperature control by the heater 541. Then, PBS is supplied to the marking flow channel 720, and the secondary cleaning step is performed.

In step S39, after the emulsion PCR treatment, a specimen containing hybridized DNAis supplied to a detection device, and the marking substance 17 binding to the DNA is detected. When the detection device is a flow cytometer, the magnetic particle containing the DNA binding to the marking substance 17 flows through a flow cell, and the magnetic particle is irradiated with a laser beam. Then, fluorescence emitted from the marking substance 17 by being irradiated with the laser beam is detected.

When a series of specimen treatment is performed using the plurality of specimen treatment chips 100A, 100B, and 100C as described above, a structure including the reservoir 750 illustrated in Fig. 46 is preferable. This is because a liquid is allowed to be easily transferred by using a pipetter in each of the following cases of: supplying a liquid after treatment by the nucleic acid extraction device to the specimen treatment chip 100C; supplying a liquid after treatment by the specimen treatment chip 100C to the specimen treatment chip 100B; and supplying a liquid after treatment by the specimen treatment chip 100B to the specimen treatment chip 100A. When there are provided the reservoir 750a for storing a liquid to be injected into the fluid module 200 and the reservoir 750b for storing a liquid to be fed from the fluid module 200 in a specimen treatment chip, it is possible to smoothly and easily perform operations of extracting a liquid after being treated, and feeding the liquid to a subsequent specimen treatment chip.

It is to be understood that the embodiments disclosed this time are examples in all respects, and are not restrictive. The scope of the present invention is indicated not by the description of the above embodiments but by the scope of claims, and includes meanings equivalent to the scope of claims and all changes (modifications) within the scope.

### REFERENCE SIGNS LIST

10: nucleic acid
11: reagent for amplification reaction of nucleic acid
12: primer
13: carrier
14: droplet
15: dispersion medium
16: reagent for breaking down droplet
17: marking substance
100, 100A, 100B, 100C: specimen treatment chip
101: port for injecting liquid
102: port for recovering liquid
110: first flow channel
111a: first channel
111b: second channel
113: intersection
120: second flow channel
130: third flow channel
132a: connection portion for allowing reagent for breaking down droplet to flow therethrough
132b: connection portion for allowing droplet containing carrier binding to amplification product of nucleic acid to flow therethrough
133: bent portion
134: linear portion
140: fourth flow channel
150: fifth flow channel
151: channel
152a, 152b: connection portion
200: fluid module
202: connection portion
300: substrate
310: through hole
500: specimen treatment apparatus
510: installation unit
520: liquid feeder
521: pump
522: valve
530: control unit
541: heater
542: magnet unit
550: detection unit
710: breaking-down flow channel
720: marking flow channel
730: droplet forming flow channel
750a: reservoir for storing liquid to be injected into fluid module
750b: reservoir for storing liquid to be fed out from fluid module

## Claims

1. A specimen treatment chip that is installed in a specimen treatment apparatus to treat a nucleic acid in a specimen supplied by the specimen treatment apparatus, the specimen treatment chip comprising:
a first flow channel for forming a droplet containing a mixed liquid of a nucleic acid, a reagent for an amplification reaction of the nucleic acid, and a carrier to which a primer for binding to the nucleic acid is added, in a dispersion medium;
a second flow channel for amplifying the nucleic acid in the droplet; and
a third flow channel for mixing the droplet containing the carrier with the primer binding to an amplification product of the nucleic acid, and a reagent for breaking down the droplet, to break down the droplet,
wherein the carrier extracted from the droplet broken down is collected to cause the amplification product on the collected carrier and a marking substance for detecting the amplification product to react with each other.

2. The specimen treatment chip according to claim 1, wherein
the first flow channel, the second flow channel, and the third flow channel are connected so that a liquid containing the nucleic acid flows continuously.

3. The specimen treatment chip according to claim 1 or 2, further comprising:
a fourth flow channel for collecting the carrier extracted from the droplet by breaking down; and
a fifth flow channel for causing the amplified product on the collected carrier and the marking substance to bind to each other.

4. The specimen treatment chip according to any one of claims 1 to 3, wherein
the first flow channel includes:
a first channel through which the mixed liquid flows;
a second channel through which the dispersion medium immiscible to the mixed liquid flows; and
an intersection at which the first channel and the second channel intersect.

5. The specimen treatment chip according to claim 4, wherein
each of the channels has a width of 5 µm or more and 100 µm or less at the intersection.

6. The specimen treatment chip according to any one of claims 1 to 5, further comprising:
a fluid module including the first flow channel, the second flow channel, and the third flow channel;
a substrate on which the fluid module is disposed,
wherein the substrate has a port for injecting a liquid into the fluid module.

7. The specimen treatment chip according to any one of claims 1 to 5, further comprising:
a plurality of the fluid modules;
a substrate on which the plurality of the fluid modules is disposed; and
a connection flow channel for connecting the plurality of the fluid modules to feed a liquid,
wherein the first flow channel, the second flow channel, and the third flow channel are individually formed in corresponding fluid modules, and
the substrate has a port for injecting a liquid into the fluid modules.

8. The specimen treatment chip according to claim 6 or 7, wherein
the port for injecting a liquid into the fluid module is a through hole formed on the substrate.

9. The specimen treatment chip according to any one of claims 6 to 8, wherein
the substrate has a thickness of 1 mm or more and 5 mm or less.

10. The specimen treatment chip according to any one of claims 6 to 9, wherein
the substrate is made of glass.

11. The specimen treatment chip according to any one of claims 6 to 10, wherein
the substrate and the fluid module are bonded by solid phase bonding.

12. The specimen treatment chip according to claim 8, wherein
the through holes are formed at predetermined pitches on the substrate.

13. The specimen treatment chip according to claim 12, wherein
the fluid module is disposed at a position suitable for the through holes formed at the predetermined pitches on the substrate, and includes connection portions connected to the corresponding through holes.

14. The specimen treatment chip according to any one of claims 1 to 13, wherein
the third flow channel has a curved shape to generate a turbulent flow for mixing the droplet and the reagent for breaking down the droplet.

15. The specimen treatment chip according to any one of claims 1 to 13, wherein
the third flow channel has a meander shape.

16. The specimen treatment chip according to any one of claims 1 to 13, wherein
the third flow channel includes a plurality of bent portions and a plurality of linear portions connecting between corresponding bent portions.

17. The specimen treatment chip according to claim 3, wherein
the carrier is a magnetic particle, and
the fourth flow channel includes a linear portion for moving back and forth the magnetic particle captured by a magnetic force in a direction along the fourth flow channel.

18. The specimen treatment chip according to claim 17, wherein
the fourth flow channel includes a connection portion to the outside for allowing a liquid containing the magnetic particle to flow in therethrough, and
the linear portion of the fourth flow channel has a flow channel width larger than a flow channel width in the connection portion.

19. The specimen treatment chip according to claim 17 or 18, wherein
the fourth flow channel includes a connection portion for supplying a cleaning liquid and a connection portion for supplying the magnetic particle, connected to one end side of the linear portion, and a connection portion for discharging the cleaning liquid and a connection portion for feeding the magnetic particle, connected to the other end side of the linear portion.

20. The specimen treatment chip according to any one of claims 1 to 19, wherein
the second flow channel is formed so that the droplet alternately passes through a plurality of temperature zones.

21. The specimen treatment chip according to claim 20, wherein
the second flow channel is formed in such a shape as to extend back and forth in the plurality of temperature zones by the number corresponding to the number of thermal cycles across the plurality of temperature zones.

22. The specimen treatment chip according to claim 3, wherein
the fifth flow channel includes one of connection portions for allowing a liquid to flow in therethrough, the other of the connection portions for allowing the liquid to flow out therethrough, and a channel connecting between the respective connection portions, and
the channel of the fifth flow channel has a channel width varying along a flowing direction of the liquid.

23. The specimen treatment chip according to any one of claims 1 to 22, further comprising:
a port for recovering a liquid passed through the first flow channel, the second flow channel, and the third flow channel.

24. A specimen treatment apparatus configured to treat a nucleic acid in a specimen by using the specimen treatment chip according to any one of claims 1 to 23, the specimen treatment apparatus comprising:
an installation unit that installs the specimen treatment chip;
a liquid feeder for supplying and feeding a liquid containing the nucleic acid to the specimen treatment chip; and
a control unit for controlling the liquid feeder so as to cause a liquid containing the nucleic acid to be fed in the specimen treatment chip while flowing through the first flow channel, the second flow channel, and the third flow channel, of the specimen treatment chip, in order.

25. The specimen treatment apparatus according to claim 24, wherein
the control unit controls the liquid feeder so as to cause a liquid containing the nucleic acid to continuously flow into the first flow channel, the second flow channel, and the third flow channel.

26. The specimen treatment apparatus according to claim 25, wherein
the liquid feeder includes a pump for controlling pressure for driving a liquid and a plurality of valves for opening and closing a pressure supply path of pressure for a liquid, and
the control unit controls opening and closing of each of the valves of the liquid feeder so as to feed the liquid in the specimen treatment chip to the first flow channel, the second flow channel, and the third flow channel by pressure.

27. The specimen treatment apparatus according to claim 26, wherein
the control unit controls supply pressure to be applied to the mixed liquid and the dispersion medium by the liquid feeder so that the mixed liquid and the dispersion medium are fed into the first flow channel by the applied pressure to form the droplet.

28. The specimen treatment apparatus according to claim 27, wherein
the control unit controls pressure of the liquid feeder so that the droplets are formed at a rate of 0.6 million pieces/minute or more and 18 million pieces/minute or less.

29. The specimen treatment apparatus according to any one of claims 24 to 28, further comprising:
a heater for forming a plurality of temperature zones in the second flow channel of the specimen treatment chip.

30. The specimen treatment apparatus according to any one of claims 24 to 29, further comprising a magnet unit,
wherein the carrier is a magnetic particle, and
the magnet unit applies a magnetic force to the magnetic particle.

31. The specimen treatment apparatus according to any one of claims 24 to 30, further comprising:
a detection unit for detecting the nucleic acid on the basis of a mark caused by the marking substance binding to the amplification product.

32. A specimen treatment method for treating a nucleic acid by using a specimen treatment chip having a first flow channel, a second flow channel, and a third flow channel, the specimen treatment method comprising the steps of:
supplying a mixed liquid of the nucleic acid, a reagent for an amplification reaction of the nucleic acid, and a carrier to which a primer for binding to the nucleic acid is added, and a dispersion medium, to the first flow channel to form a droplet;
supplying an emulsion containing the droplet to the second flow channel to amplify the nucleic acid;
supplying the droplet containing the carrier with the primer binding to an amplification product of the nucleic acid, and a reagent for breaking down the droplet, to the third flow channel to break down the droplet; and
collecting the carrier extracted from the droplet broken down to cause the amplification product on the collected carrier and a marking substance for detecting the amplification product to react with each other.

33. A specimen treatment chip configured to be installed in a specimen treatment apparatus to treat a nucleic acid in a specimen, the specimen treatment chip comprising:
a breaking-down flow channel for mixing a droplet containing a carrier binding to an amplification product of the nucleic acid, amplified in the droplet, and a reagent for breaking down a droplet, to break down the droplet.

34. The specimen treatment chip according to claim 33, wherein
the breaking-down flow channel includes a connection portion for allowing the droplet to flow therethrough, and a connection portion for allowing a reagent for breaking down the droplet to flow therethrough.

35. The specimen treatment chip according to claim 33 or 34, further comprising:
a marking flow channel for collecting the carrier extracted from the droplet by breaking down and causing the amplification product on the extracted carrier and a marking substance for detecting the amplification product to bind to each other.

36. The specimen treatment chip according to claim 35, wherein
the breaking-down flow channel and the marking flow channel are connected so that a liquid containing the nucleic acid flows continuously.

37. The specimen treatment chip according to any one of claims 33 to 36, wherein
the breaking-down flow channel has a curved shape for mixing the droplet and a reagent for breaking down the droplet.

38. The specimen treatment chip according to any one of claims 33 to 36, wherein
the breaking-down flow channel has a meander shape.

39. The specimen treatment chip according to any one of claims 33 to 36, wherein
the breaking-down flow channel includes a plurality of bent portions and a plurality of linear portions connecting between the corresponding bent portions.

40. The specimen treatment chip according to any one of claims 33 to 39, wherein
the breaking-down flow channel is provided on its inner wall with a plurality of protrusions for mixing the droplet and the reagent for breaking down the droplet.

41. The specimen treatment chip according to claim 35, wherein
the carrier is a magnetic particle, and
the marking flow channel includes a linear portion for moving back and forth the magnetic particle captured by a magnetic force in a direction along the marking flow channel.

42. The specimen treatment chip according to claim 41, wherein
the marking flow channel includes a connection portion for allowing a liquid containing the magnetic particle extracted from the droplet by breaking down to flow in therethrough, and
the linear portion of the marking flow channel has a flow channel width larger than a flow channel width in the connection portion.

43. The specimen treatment chip according to claim 35, wherein
the breaking-down flow channel is further provided on its one end side with a connection portion for supplying a cleaning liquid and a connection portion for supplying the marking substance, and
the marking flow channel is provided on its the other end side with a connection portion for feeding a carrier in which the marking substance has reacted with the amplified product on the carrier, and a connection portion for discharging the cleaning liquid.

44. The specimen treatment chip according to claim 35, further comprising:
a fluid module including the breaking-down flow channel and the marking flow channel; and
a substrate on which the fluid module is disposed,
wherein the substrate includes at least one of a port for injecting a liquid into the fluid module and a port for feeding a liquid from the fluid module.

45. The specimen treatment chip according to claim 44, wherein
at least one of the port for injecting a liquid into the fluid module and the port for feeding a liquid from the fluid module is a through hole formed on the substrate.

46. The specimen treatment chip according to claim 44 or 45, further comprising:
a reservoir for storing a liquid to be injected into the fluid module or a reservoir for storing a liquid to be fed from the fluid module, provided over the port.

47. The specimen treatment chip according to claim 46, wherein
the reservoir is provided in its upper portion with an opening larger than a diameter of the port.

48. The specimen treatment chip according to any one of claims 44 to 47, wherein
the substrate has a thickness of 1 mm or more and 5 mm or less.

49. The specimen treatment chip according to any one of claims 44 to 48, wherein
the substrate is made of glass.

50. The specimen treatment chip according to any one of claims and 44 to 48, wherein
the substrate is made of resin composition.

51. The specimen treatment chip according to any one of claims and 44 to 50, wherein
the substrate and the fluid module are bonded by solid phase bonding.

52. The specimen treatment chip according to claim 45, wherein
the through holes are formed at predetermined pitches on the substrate.

53. The specimen treatment chip according to claim 52, wherein
the fluid module is disposed at a position suitable for the through holes formed at the predetermined pitches on the substrate, and includes connection portions connected to the corresponding through holes.

54. A specimen treatment apparatus for treating a nucleic acid in a specimen by using the specimen treatment chip according to any one of claims 33 to 53, the specimen treatment apparatus comprising:
an installation unit that installs the specimen treatment chip;
a liquid feeder for supplying and feeding a liquid containing the nucleic acid to the specimen treatment chip; and
a control unit for controlling the liquid feeder so as to cause the liquid containing the nucleic acid to be fed in the specimen treatment chip while flowing through the breaking-down flow channel of the specimen treatment chip.

55. The specimen treatment apparatus according to claim 54, wherein
the specimen treatment chip includes a marking flow channel configured to collect the carrier extracted from the droplet by breaking down to cause the amplification product on the collected carrier and a marking substance for detecting the amplification product to bind to each other, and
the control unit controls the liquid feeder so as to cause the liquid containing the nucleic acid to continuously flow in the breaking-down flow channel and the marking flow channel.

56. The specimen treatment apparatus according to claim 54, wherein
the control unit controls the liquid feeder so as to cause the liquid containing the nucleic acid to flow back and forth in the breaking-down flow channel along the flow channel.

57. The specimen treatment apparatus according to any one of claims 54 to 56, wherein
the liquid feeder includes a pump for controlling pressure for driving a liquid and a plurality of valves for opening and closing a pressure supply path of pressure for a liquid, and
the control unit controls opening and closing of each of the valves of the liquid feeder so as to feed the liquid in the specimen treatment chip to the breaking-down flow channel by pressure.

58. The specimen treatment apparatus according to claim 54, further comprising a heater,
wherein the specimen treatment includes a marking flow channel configured to collect the carrier extracted from the droplet by breaking down to cause the amplification product on the collected carrier and a marking substance for detecting the amplification product to bind to each other, and
the heater is disposed at a position overlapping with the marking flow channel of the specimen treatment chip.

59. The specimen treatment apparatus according to claim 58, further comprising a magnet unit in the marking flow channel of the specimen treatment chip,
wherein the carrier is a magnetic particle, and
the magnet unit applies a magnetic force to the magnetic particle.

60. The specimen treatment apparatus according to claim 59, wherein
the magnet unit is disposed on a side opposite to the heater across the marking flow channel of the specimen treatment chip.

61. A specimen treatment method for treating a nucleic acid by using a specimen treatment chip, the specimen treatment method comprising the steps of:
forming a droplet containing a mixed liquid of the nucleic acid, a reagent for an amplification reaction of the nucleic acid, and a carrier to which a primer for binding to the nucleic acid is added, in a dispersion medium;
amplifying the nucleic acid in the droplet; and
supplying the droplet containing the carrier with the primer binding to an amplification product of the nucleic acid, and a reagent for breaking down the droplet, to the breaking-down flow channel provided the specimen treatment chip to break down the droplet.

62. The specimen treatment method according to claim 61, wherein
the specimen treatment chip includes a marking flow channel, and
the carrier extracted from the droplet by breaking down in the breaking-down flow channel is supplied to the marking flow channel to cause the amplification product on the carrier and a marking substance for detecting the amplification product to react with each other.

63. The specimen treatment method according to claim 61, wherein
a mixed liquid of the nucleic acid, a reagent for an amplification reaction of the nucleic acid, and the carrier, and the dispersion medium are supplied to a droplet forming flow channel provided in a specimen treatment chip different from the specimen treatment chip provided with the breaking-down flow channel to form the droplet.

64. The specimen treatment method according to any one of claims 61 to 63, wherein
the droplet and the reagent for breaking down the droplet are supplied into the breaking-down flow channel, and
the reagent for breaking down the droplet is caused to flow back and forth in the breaking-down flow channel to break down the droplet.
